# EUROPEAN PATENT SPECIFICATION

(11) **EP 3 172 337 B1**
(45) Date of publication and mention of the grant of the patent: **01.04.2020**
(21) Application number: 15738711.9
(22) Date of filing: 22.07.2015
(51) Int. Cl.: C12Q 1/689, C12Q 1/6895

(54) **METHOD FOR THE DETECTION OF SEPSIS**
VERFAHREN ZUM NACHWEIS VON SEPSIS
PROCÉDÉ POUR LA DÉTECTION DE SEPSIS

(30) Priority: 23.07.2014 EP 14178228; 11.11.2014 EP 14192644
(43) Date of publication of application: 31.05.2017
(73) Proprietor: CONGEN Biotechnologie GmbH, 13125 Berlin (DE)
(72) Inventor: MERGEMEIER, Steffen, 10243 Berlin (DE)
(74) Representative: Hertin und Partner Rechts- und Patentanwälte PartG mbB
(86) International application number: PCT/EP2015/066779
(87) International publication number: WO 2016/012508

(56) References cited:
- WO-A1-96/15267
- WO-A1-2009/000430
- WO-A2-01/77392
- WO-A2-99/58713
- US-A1- 2007 218 489
- US-A1- 2008 118 923
- US-A1- 2009 061 446
- US-A1- 2012 024 701
- TOMASZ GOSIEWSKI ET AL: "A novel, nested, multiplex, real-time PCR for detection of bacteria and fungi in blood", BMC MICROBIOLOGY, BIOMED CENTRAL LTD, GB, vol. 14, no. 1, 4 June 2014 (2014-06-04), page 144, XP021187371, ISSN: 1471-2180, DOI: 10.1186/1471-2180-14-144
- ÁDÁM HORVÁTH ET AL: "A novel, multiplex, real-time PCR-based approach for the detection of the commonly occurring pathogenic fungi and bact", BMC MICROBIOLOGY, BIOMED CENTRAL LTD, GB, vol. 13, no. 1, 23 December 2013 (2013-12-23), page 300, XP021172036, ISSN: 1471-2180, DOI: 10.1186/1471-2180-13-300

## Description

The invention relates to an in vitro method for the detection and/or identification of a human pathogen and/or genetic material thereof comprising subjecting a sample comprising or suspected of comprising a human pathogen and/or genetic material thereof to a multiplex real-time polymerase chain reaction (RT-PCR), wherein said method comprises amplification of PCR products that enable discrimination between bacterial and fungal pathogens, discrimination between gram positive and gram negative bacterial pathogens, and identification of Staphylococcus and/or Candida pathogens if present in said sample. The invention also relates to the oligonucleotides used in said method and a set of oligonucleotides and a kit for carrying out the method.

### BACKGROUD OF THE INVENTION

Sepsis is presently understood as an invasion of microorganisms and/or their toxins in the blood stream of a patient in combination with the immune reaction of the infected patient towards the invasion. The term "sepsis" has long been used, whereby the modern definition of a sepsis was developed in the early 19^{th} century, when it was discovered that pathogenic bacteria in the blood system of a patient lead to the disease.

Despite having long been recognized as a dangerous disease, approximately 60,000 cases lead to death per year in Germany alone. After heart attack, sepsis represents the third most common cause of death and the seventh most common diagnosis of a life-endangering disease. In 2011, 87,000 patients were diagnosed with sepsis, 69,000 with a severe sepsis and 19,000 with septic shock. In approximately 70 % of the cases a nosocomial infection was identified as the cause. 10.5 % of patients with a sepsis and 42.8 % of patients with a severe sepsis ultimately died of the disease. One reason for the high rate of death is that diagnosis of the condition is often too late for an effective course of antibiotics.

Sepsis can be defined as sepsis, severe sepsis or septic shock. The basis for the different diagnoses is provided by the American College of Chest Physicians (ACCP) in the Society of Critical Care Medicine (SCCM). Sepsis is diagnosed when an infection is present or likely and at least two criteria of a Systemic Inflammatory Response Syndrome (SIRS) are present. SIRS symptoms relate to fever over 38 ° C, increased heart rate above 90 beats per minute, Tach-ypnea with a frequency of ≤ than 20/min, hyperventilation, a leucocytosis (≥ than 12.000/mm³) or a leucopenia (≤ 4.000/mm³). When in addition to these criteria additional organ dysfunction such as an acute thrombocytopenia or arterial hypoxia or renal dysfunction are identified, then a severe sepsis can be diagnosed. The most serious form of the disease is a septic shock, which is defined by a 40-60 % rate of death and is characterized by an untreatable breakdown in circulation.

Despite the clear criteria for diagnosis and for differentiation between the various forms of the disease, a diagnosis is often only provided too late for effective treatment.

In Germany and North America the most common causes of a sepsis are bacteria such as E.coli, Staphylococcus aureus or Pneumokokken. In addition to bacterial infection, fungal infection has in recent times become a significant cause of the disease. Candida infections are the third most common cause of infection after Staphylococcus epidermis and Staphylococcus aureus in the USA. Fungal infections tend to be associated with higher rates of death, in some cases as for Aspergillus fumigatus up to 90 %. Only approximately 5 % of fungal caused cases of sepsis are identified during the disease due to the poor diagnostic methods available.

Recent studies have shown that patients with severe sepsis or septic shock showed an increased likelihood of death of 7.6 % for every hour in which antibiotic therapy is not applied. Survival rates are also significantly reduced when antibiotics are not applied within the first 6 hours of identifying hypotension. Survival rates will be significantly increased if diagnosis times are reduced.

Culturing microorganisms from blood samples remains the gold standard in detection of the microbiological cause of sepsis. This method is however subject to significant disadvantages, in particular due to the large time difference between taking a blood sample and providing the results. It is not uncommon that 24 to 72 hours pass between taken a sample and providing diagnostic information. Within this time broad band untargeted antibiotic therapies are often introduced. This may lead to some success in treating the disease but is related to significant disadvantages with respect to the development of multi resistant microorganisms.

Further disadvantages of a culture method are the poor sensitivity and the large amount of blood required for diagnosis. Additionally the risk of contamination is relatively high during blood sampling.

Alternative methods are required. In particular, methods based on the polymerase chain reaction (PCR) have been proposed. The major advantage of PCR based methods is their speed, whereby diagnostically relevant information can be provided within a number of hours and an appropriate antibiotic therapy may subsequently be introduced. The high sensitivity and small sample size are also beneficial features of the PCR method.

A number of PCR based methods for the detection of sepsis are available on the market, for example the multiplex PCR assay VYOO® from SIRS-Lab, the LightCycler® SeptiFast from Roche or the SeptiTest® from Molzym. Such tests are able to provide identification of bacterial infection by general detection of the 16S ribosomal gene of the bacteria. The costs for such methods are however relatively high at around 150-250 EUR per reaction. Microarray approaches have also been described. Microarrays require however a significant amount of time in effort and may not provide detection of any given human pathogen.

Alternative diagnostic methods presently relate to various biomarkers for sepsis. A standard parameter for sepsis diagnosis relates to determination of C-reactive protein (CRP) protein levels. For example, ELISA tests based on PCT detection enable a reliable identification of the disease. Additional biomarkers for sepsis have been identified, which may be detected via various technical means, for example S100B (US 7,569,209), ca125 (US 7,517,518) or CA 19-9 (US 7,572,590). Such approaches may provide reasonable identification of a sepsis, but do not provide the necessary information on the pathogenic cause of the disease, which is essential for providing effective antibiotic treatment.

Microarray and multiplex PCR approaches have been disclosed in the art, which are typically defined by extremely large numbers of probes or primers required for application in such methods (leading to significant cost and effort), a limited pool of target pathogens capable of being detected (such as only a limited sub-group of bacterial pathogens, or the absence of fungal pathogens), or a lack of discrimination between gram negative and gram positive bacterial pathogens, which provides sub-standard information for appropriate antibiotic therapies (US 2009286691 and US 2011151453). Methods for discriminating gram-positive and gram-negative bacteria have been disclosed in the art (US 20080118923 A1), in addition to the combined analysis of 16S and 18S sequences of bacteria and fungi (US 20090061446). Such methods, although potentially useful in clinical diagnostics, have never been applied in sepsis analytics and employ large numbers of primers in either microarray or very complex multiplex reactions, representing a significant technical and financial challenge for clinical diagnostic laboratories.

Multiplex RT-PCR approaches have been described in which a number of commonly occurring human pathogens are potentially detected. One example of such a multiplex PCR method is described in Gosiewski et al (BMC Microbiology 2014, 14:144), which discloses a nested PCR approach for detecting gram positive and gram negative bacteria, yeast fungi and filamentous fungi from blood samples. A nested polymerase chain reaction involves two sets of primers, used in two successive runs of polymerase chain reaction, the second set intended to amplify a secondary target within the first run product. Nested PCR is applied in order to reduce nonspecific binding in products due to the amplification of unexpected primer binding sites, as it is unlikely that any of the unwanted PCR products contain binding sites for both the new primers in the second run, ensuring the product from the second PCR has little contamination from unwanted products of primer dimers, hairpins, and alternative primer target sequences. Despite potentially reducing background signal, the PCR method described in Gosiewski is relatively complex and requires two cycling reactions, essentially doubling the time, effort and reagents required for the analysis. The requirement of running two PCR reactions in a clinical diagnostics laboratory is severely disadvantageous and also indicates that the authors of Gosiewski were unable to develop a multiplex PCR method using 16S and 18S rRNA sequences that was sufficiently sensitive and specific to be processed in a single cycling run.

Other methods have employed the amplification of a number of PCR products from bacterial and fungal pathogens using sequence-specific oligonucleotides together with sequence-unspecific dyes, and subsequently a melting curve analysis to differentiate between the various products (Horvath et al, BMC Microbiology 2013, 13:300). The method disclosed therein is however limited by a number of disadvantages known to occur with melting curve analyses. Differentiation of PCR products is in theory possible but in practical circumstances very difficult to carry out, and as the authors state, is not possible for various pathogens due to very similar amplified sequences.

The 16S and 18S rRNA sequences have been previously identified as PCR amplification targets for identifying bacteria. For example, WO 99/58713 describes a method for the detection of microorganisms, in which the 16S region has been identified as an amplification target. The method described therein is however directed primarily towards detection of E. coli in pharmaceutical or cosmetic products. WO 99/58713 makes no mention of the application of such a target sequence in the detection of a large number of human pathogens, for example in a diagnostic kit for sepsis. The state of the art therefore demonstrates a need for improved means for detecting and/or identifying human pathogens, for example those associated with sepsis. Previous methods are commonly limited by the number of pathogens that may potentially be detected, and to limitations in pathogen detection due to the fallibilities of older analyses techniques, such as melting curve analyses, or the use of large numbers of primers, thereby increasing the risk of unwanted background signal and difficulties in analysis.

### SUMMARY OF THE INVENTION

In light of the disadvantages of the prior art the technical problem underlying the invention was the provision of means for the fast and reliable detection of a pathogen from a sample in order to enable the provision of appropriate means for treating said pathogen.

The present invention therefore seeks to provide a method, kit and further means for diagnosis of sepsis and/or detection and/or identification of a pathogen on the basis of a nucleic acid amplification method, oligonucleotides and other reagents.

The solution to the technical problem of the invention is provided in the independent claims. Preferred embodiments of the invention are provided in the dependent claims.

The present invention therefore provides a in a preferred embodiment a sensitive multiplex RT-PCR amplification method for the detection of bacteria and fungi.

The invention relates to An in vitro method for the detection and/or identification of a human pathogen and/or genetic material thereof comprising subjecting a sample comprising or suspected of comprising a human pathogen and/or genetic material thereof to a multiplex real-time polymerase chain reaction (RT-PCR), wherein said method comprises amplification of PCR products that enable:
a) discrimination between bacterial and fungal pathogens,
b) discrimination between gram positive and gram negative bacterial pathogens, and
c) identification of Staphylococcus and/or Candida pathogens if present in said sample,
wherein the method is carried out in a single PCR reaction using oligonucleotide primers and probes that hybridize with a 16S rRNA sequence of a bacterial pathogen and a 18S rRNA sequence of a fungal pathogen, wherein said probes exhibit distinct fluorescent labels that may be identified and differentiated from one another on the basis of emitting light at different wavelengths, and
wherein the oligonucleotide primers that hybridize with a 16S rRNA sequence hybridize with and prime the amplification of a sequence that corresponds to SEQ ID No. 13 with reference to the Escherichia coli 16S sequence, and the oligonucleotide primers that hybridize with an 18S rRNA sequence hybridize with and prime the amplification of a sequence that corresponds to SEQ ID No. 14 with reference to the Candida albicans 18S sequence.

This technical solution solves the problem stated above, namely it enables the detection and/or identification of a human pathogen, thereby enabling informed decisions to be made regarding appropriate treatment, for example of a patient exhibiting symptoms of sepsis or similar blood infections.

The invention provides two PCR systems combined for enhanced sepsis detection. The application of real time PCR using a multiplex PCR method enables bacteria and fungus to be detected. The method is based on detection of the 16S rRNA gene of bacteria and the 18S rRNA gene of fungus. These systems are used in combination. Additional probes for actinobacteria and firmicutes are provided, which enable a differentiation between gram-positive and gram-negative bacteria, which ultimately plays a crucial role in selection of an appropriate antibiotic therapy.

Probes are provided for the 16S rRNA genes, probes for general firmicuten detection, including additional probes for clostridien detection and mycoplasmen detection and probes are provided for actinobacteria detection. Probes are also provided for the 18S rRNA gene.

The present invention allows identification and/or detection of a vast number of human pathogenic bacteria, the differentiation between gram-positive and gram-negative bacteria and the detection of staphylococcus as the most common gram-positive pathogen. A vast number of human pathogenic fungi are also able to be detected, in particular the detection of Candida as the most common fungal pathogen.

The method of the invention is carried out in a single multiplex real-time polymerase chain reaction (RT-PCR), otherwise known as a single PCR run. The term "single reaction" or "single run" in this context refers to a standard operating programme used by RT-PCR devices, typically comprising a series of 20 to 40 repeated temperature changes, called cycles, with each cycle commonly consisting of 2 to 3 discrete temperature steps, often referred to as denaturation, annealing and elongation steps. The cycling is often preceded by a single temperature step at a high temperature (>90 °C), and followed by one hold temperature at the end of the reaction for final product extension or brief storage. By way of example, without being limiting, the "single reaction" therefore refers preferably to one PCR reaction, preferably carried out in a single PCR vessel or test tube, that is typically subjected to one programme of 20 to 40 temperature cycles to amplify the desired product.

The present invention surprisingly shows that the combined analysis of the 16S rRNA gene of bacteria and the 18S rRNA gene of fungi is possible in a single PCR reaction, preferably without a nested PCR approach, in such a manner that a highly sensitive and very specific method is provided. Previous attempts at providing such a combined PCR had been bothered by background signal caused by unwanted products of primer dimers, hairpins, and alternative primer target sequences. The present invention is, according to the inventor, the first described single-run RT-PCR multiplex method for discrimination between bacterial and fungal pathogens, discrimination between gram positive and gram negative bacterial pathogens, and identification of Staphylococcus and/or Candida pathogens if present in said sample.

In a preferred embodiment the method as described herein is characterised in that one primer set, said set comprising two primers (a forward primer and a reverse primer), is used to amplify a PCR product for each of the 16S and 18S regions. In a preferred embodiment the method is therefore characterised in that two primer sets, namely one for the 16S region and one for 18S region, are applied in the method.

The low number of primers required to detect the vast number of potential pathogens represents a significant advantage for clinical diagnostics. The low number of primers required for the multiplex reaction reduces the likelihood or risk of primer dimer formation or errors occurring during oligonucleotide synthesis. The complexity of the present method is significantly reduced compared to alternative amplification schemes described previously, thereby increasing the user friendliness and reproducibility compared to those methods of the prior art.

It was entirely unexpected that a multiplex PCR system could be designed, in which only two sets of primers are applied for 16S and 18S amplification, in combination with multiple sequence-specific probes, which enable a highly sensitive method capable of detecting a vast number of potential human pathogens in a simple and reliable multiplex setup that exhibits no significant disadvantages with respect to unwanted background signal.

In other embodiments the number of primer sets used for 16S or 18S amplification may be 1 for each region (e. g. the 16S region), or 2, 3, 4 or 5 primer sets for each region, preferably with corresponding probes designed to identify the amplification products. The invention is therefore characterised by the reduced number of primer sets employed in the method compared to the prior art.

The method is characterised in that the RT-PCR reaction comprises oligonucleotides that bind a 16S rRNA sequence of a bacterial pathogen.

The method is characterised in that the RT-PCR reaction comprises oligonucleotides that bind an 18S rRNA sequence of a fungal pathogen.

The use of the 16S and 18S regions in the multiplex RT-PCR reaction described herein is particularly advantageous and represents an improvement over the art. Similar systems have employed analysis of the ITS1 or ITS2 genes, which are not as effective in determining bacterial/fungal differences or providing sensitive results. Amplification of the ITS sequences has proven more difficult and unreliable than the 16S or 18S sequences as presently claimed.

The method is characterised in that the oligonucleotides that bind a 16S rRNA sequence of a bacterial pathogen bind a sequence that corresponds to SEQ ID No. 13 with reference to the Escherichia coli 16S sequence.

The method is characterised in that the oligonucleotides that bind an 18S rRNA sequence of a fungal pathogen bind a sequence that corresponds to SEQ ID No. 14 with reference to the Candida albicans 18S sequence.

The amplification and/or analyses of these particular regions of the genomes of bacterial and fungal pathogens has been neither suggested not disclosed in the art and is associated with unexpected benefits. Through the amplification of these regions a greater number of pathogens may be amplified in comparison to those systems described in the art. These regions are of sufficient sequence similarity amongst the various pathogens to enable amplification of essentially all known bacterial and fungal pathogens. These particular sequence regions of the 16S and 18S genes have not been previously identified for such application. The term "a sequence that corresponds to SEQ ID No. 13 with reference to the Escherichia coli 16S sequence" represents any sequence of any given human bacterial pathogen that shows sufficient sequence similarity to be amplified in the method described herein. The phrase covers corresponding sequences in other bacteria that, although the numbering may or may not be the same as for the E. coli gene, still comprise of a corresponding sequence in the 16S region that comprises a sequence to be amplified in the method.

The term "a sequence that corresponds to SEQ ID No. 14 with reference to the Candida albicans 18S sequence" represents any sequence of any given human fungal pathogen that shows sufficient sequence similarity to be amplified in the method described herein. The phrase covers corresponding sequences in other fungi that, although the numbering may or may not be the same as for the Candida gene, still comprise of a corresponding sequence in the 18S region that comprises a sequence to be amplified in the method.

These regions of the 16S and 18S rRNA sequences are advantageous due to the ability to use only one set of primers for each of the 16S and 18S sequences, respectively, combined with one or more sequence specific probes for each region, and maintain the ability for detecting a vast number of potential human pathogens. The prior art has not previously identified these regions as being suitable for this purpose in sepsis diagnostics.

In a preferred embodiment the method of the invention is characterised in that the sample obtained from a subject exhibiting one or more symptoms of systemic inflammatory response syndrome (SIRS), sepsis, severe sepsis and/or septic shock. The invention therefore provides amplification method for the detection and/or identification of bacteria and fungi during diagnosis and/or identification of the causative pathogen of sepsis.

A significant benefit of this approach is the ability to subsequently prescribe an appropriate medicament during treatment. In light of the knowledge regarding bacterial, gram-positive, gram-negative or fungal pathogen presence, an appropriate antibiotic can be selected for treatment, thereby avoiding potentially useless antibiotic treatments and associated financial, health and environmental disadvantages.

The method of the invention is characterised in that the oligonucleotides capable of binding a 16S rRNA sequence of a bacterial pathogen enable discrimination between gram positive and gram negative bacteria.

In one embodiment the method of the invention is characterised in that the oligonucleotides that hybridize with a 16S rRNA sequence of a bacterial pathogen comprise the oligonucleotides of a) and b), or c), wherein:
a) Oligonucleotide probes, each of whose sequence consists of a sequence according to SEQ ID NO 3, 4, 5, 6, 7 or 8, respectively, or oligonucleotides of a complementary sequence, wherein said probes are labelled such to be distinguished from each other,
b) Oligonucleotide primers, each of whose sequence consists of a sequence according to SEQ ID NO 1 or 2, respectively, or oligonucleotides of a complementary sequence,
c) Oligonucleotides of a) and b) that comprise a 0 to 5 nucleotide addition or deletion at their 5' and/or 3' end, or oligonucleotides of a complementary sequence.

In a surprising manner, the oligonucleotides designed for 16S rRNA amplification (which may be referred to as the bacterial amplification system of the invention) are able to amplify genetic material from a vast number of pathogenic or potentially pathogenic bacteria, despite sequence variation of this region between the many bacterial species. The relatively small number of oligonucleotides enables a simple and straightforward multiplex approach, which shows extremely good coverage in a broad-band detection method.

In one embodiment the method of the invention is characterised in that the oligonucleotides that hybridize with an 18S rRNA sequence of a fungal pathogen comprise the oligonucleotides of a) and b), or c), wherein:
a) Oligonucleotide probes, each of whose sequence consists of a sequence according to SEQ ID NO 11, 15 or 12, respectively, or oligonucleotides of a complementary sequence, wherein said probes are labelled such to be distinguished from each other,
b) Oligonucleotide primers, each of whose sequence consists of a sequence according to SEQ ID NO 9 or 10, respectively,
c) Oligonucleotides of a) and b) that comprise a 0 to 5 nucleotide addition or deletion at their 5' and/or 3' end, or oligonucleotides of a complementary sequence.

In a surprising manner, the oligonucleotides designed for 18S rRNA amplification (which may be referred to as the fungal amplification system of the invention) are able to amplify genetic material from a vast number of pathogenic or potentially pathogenic fungi, despite sequence variation of this region between the various species. The relatively small number of oligonucleotides enables a simple and straightforward multiplex approach, which shows extremely good coverage in a broad-band detection method.

In one embodiment the method of the invention is characterised in that a combination of primers and probes according to the bacterial amplification system and the fungal amplification system is provided.

The method of the invention is characterised in that the oligonucleotide probes exhibit fluorescent labels, wherein each probe may be identified and differentiated from one another on the basis of distinct fluorescent labels that emit light at different wavelengths from each other. As described in more detail below, probes and labels may be selected as required depending on the device used for analysis and the sample to be assessed.

In one embodiment the method of the invention comprises or consists of the following steps:
a) Providing a sample, preferably a blood, serum or plasma sample, preferably obtained from a subject exhibiting one or more symptoms of systemic inflammatory response syndrome (SIRS), sepsis, severe sepsis and/or septic shock,
b) Bringing said sample into contact with a mixture of oligonucleotides according to any one of the preceding claims in combination with means for carrying out a nucleic acid amplification reaction, such as amplification reaction reagents,
c) Carrying out a nucleic acid amplification reaction, preferably an RT-PCR, and
d) Detecting and evaluating the amplification products generated as a result of said nucleic acid amplification reaction.

In some embodiments the method encompasses the obtaining of a sample. The method may however also encompass merely the analysis of a sample having already been obtained. The method may in one embodiment accompany selection of a particular antibiotic for treatment of a sepsis based on the results of the identification and/or detection enabled by the method of the present invention. The method may however not encompass such antibiotic selection.

In general, the method is suitable for detection of a bacteria or fungi from a sample, this may also include identification of the bacteria and/or fungi, or partial identification, depending on the results obtained. The identification of a pathogen may occur such that the read-out of the assay provides "bacterial", "fungal", "gram-positive or gram-negative" as an appropriate result. Based on the specific probes described herein, more specific identification of the presence of a Candida species and/or the presence of a Staphylococcus species may also be provided.

In one embodiment the method of the invention is characterised in that amplification products are generated as a result of said nucleic acid amplification reaction and the detecting and/or evaluating of said products comprises:
a) detecting an amplification product, preferably of 144bp, produced using primers according to SEQ ID NO 1 and 2,
b) detecting an amplification product, preferably of 92bp, produced using primers according to SEQ ID NO 9 and 10, and
c) detecting one or more labels associated with one or more probes according to SEQ ID NO 3, 4, 5, 6, 7, 8, 11 and/or 12, wherein
   i. detection of a label associated with SEQ ID NO 3 indicates the presence of bacteria,
   ii. detection of a label associated with SEQ ID NO 4 indicates the presence of gram-positive bacteria,
   iii. detection of a label associated with SEQ ID NO 5 indicates the presence of gram-positive bacteria,
   iv. detection of a label associated with SEQ ID NO 6 indicates the presence of gram-positive bacteria,
   v. detection of a label associated with SEQ ID NO 7 indicates the presence of gram-positive bacteria,
   vi. detection of a label associated with SEQ ID NO 8 indicates the presence of a Staphylococcus species,
   vii. detection of a label associated with SEQ ID NO 11 indicates the presence of fungi,
   viii. detection of a label associated with SEQ ID NO 15 indicates the presence of fungi, and/or
   ix. detection of a label associated with SEQ ID NO 12 indicates the presence of a Candida species.

In a further aspect of the invention an oligonucleotide set is provided, comprising the oligonucleotides of a) or b), and c) or d), wherein:
a) Oligonucleotides, each of whose sequence consists of a sequence according to SEQ ID NO 1, 2, 3, 4, 5, 6, 7 or 8, respectively, or oligonucleotides of a complementary sequence,
b) Oligonucleotides of a) that comprise a 0 to 5 nucleotide deletion at their 5' and/or 3' end, or oligonucleotides of a complementary sequence,
c) Oligonucleotides, each of whose sequence consists of a sequence according to SEQ ID NO 9, 10, 11, 15 or 12, respectively, or oligonucleotides of a complementary sequence,
d) Oligonucleotides of c) that comprise a 0 to 5 nucleotide deletion at their 5' and/or 3' end, or oligonucleotides of a complementary sequence.

The nucleotide set is to be understood as either a combined, or a spatially separated set of oligonucleotides that may be easily applied in a method, for example as described herein. The oligonucleotide set relates to the bacterial amplification system and the fungal application system.

Also disclosed herein are one or more isolated oligonucleotides selected from the group consisting of:
a) Oligonucleotide with a sequence according to SEQ ID NO 1, 2, 3, 4, 5, 6, 7, 8, 9, 10, 11, 15 or 12,
b) Oligonucleotide of a) that comprise a 0 to 5 nucleotide addition or deletion at the 5' and/or 3' end of a sequence according to SEQ ID NO 1, 2, 3, 4, 5, 6, 7, 8, 9, 10, 11, 15 or 12, and/or
c) Oligonucleotide of more than 80%, 85%, 90% or preferably more than 95% sequence identity to a) or b), and/or
d) Oligonucleotides of a complementary sequence of a) to c).

Each of the oligonucleotides provided herein has been specifically developed for application in the method as described herein and to the applicant's knowledge have not been previously described.

The invention also provides a kit suitable for carrying out the method of any one of the preceding claims, comprising
a) an oligonucleotide set of the invention, and
b) amplification reagents such as one or more fluorescent labels, dNTPs, buffers and/or enzymes.

The kit as described herein may be understood as a combination of components, preferably contained however within separate containers, but within close proximity to one another. The kit may be provided with or without specific PCR reagents. In one embodiment the kit may encompass all those reagents necessary for carrying out an RT-PCR, including preferably pre-labelled probes, buffers, enzymes, dNTPs and any further reagents. The kit may however also comprise primarily of the oligonucleotide sets described herein.

In combination these methods, systems, oligonucleotides and kits enable a multiplex PCR method which is sufficiently sensitive to provide effective detection and/or identification of an infectious agent associated with sepsis.

In a preferred embodiment the method as described herein comprises the use of one or more sequence-specific probes coupled to a label capable of detection in a standard RT-PCR device. As described above, the sole use of a melting curve analysis or the identification of human pathogen is during sepsis diagnostics is hampered by significant disadvantages, primarily due to the fact that a mouth curve analysis is not sufficient to identify very minor differences in sequence that may provide important information on the particular pathogen responsible for the illness. The present invention is therefore preferably characterised by use of sequence-specific probes.

A melting curve analysis may however be combined with the method as described herein in order to provide additional information on potential background signal. In one embodiment the method comprises a) the use of a double strand specific label and b) a melting curve analysis to identify background signal, such as primer-dimer or unwanted PCR product signal. Suitable double strand specific labels are known in the art. The invention does not rely on the melting temperature analysis of multiplex PCR products; however such technology may be applied in combination with the other features of the invention described herein to potentially provide an improved or more complete analysis.

In a preferred embodiment the additional treatment of the PCR mastermix enables increases in sensitivity. In one embodiment the method of the invention therefore comprises treatment of the amplification reaction reagents to remove or neutralise genetic material capable of producing unwanted background nucleic acid amplification.

The invention therefore relates to a method for neutralising, removing or modifying genetic material capable of producing unwanted background nucleic acid amplification, so that said genetic material cannot, or can only to a limited extent enable amplification of said genetc material under any given amplification conditions, by treatment of amplification reaction reagents in solution, before addition of the oligonucleotide probes or primers with suitable reagents. Suitable reagents are known in the art, such as DNA intercalating or similar substances, that remove the possibility of background DNA being amplified.

The sensitive detection of fungus and bacteria is important in a number of potential applications. Screening for the presence of sepsis is one example of an application in clinical diagnostics. In addition, quality control of pharmaceutical products, in order to ensure microorganism free medical products, is an additional field in which the invention may be applied. In essence, any examination of sterile (or potentially sterile) medium for potential contamination with microorganisms is an appropriate field of application for the present invention. For example bodily fluids, which are in healthy patients sterile, may be assessed, such as body joint fluid, cerebral fluid or urine.

As described above, the present doctrine in sepsis definition characterizes sepsis as the invasion of a microorganism in addition to an overreaction of the immune system. No personalized predictive factors are known. The risk for patients in intensive care is very high that sepsis may occur. Because the invasive microorganism that causes the sepsis ultimately leads to a pathologic immune reaction, the maintenance of the microorganism itself in the blood is very low. In only 40 % of all sepsis patients is the causative factor detected during the illness. Because the survival rates of a patient are significantly higher with an earlier administration of antibiotic therapies, the earliest possible detection of a microorganism is desired. The detection means must be extremely sensitive.

PCR methods are already known for their high sensitivity and speed during sepsis screening. One significant problem with PCR applications is the detection of at least one or some individual microorganisms or fungi of potentially any given bacterial and fungal population from a sample, whilst differentiating pathogenic bacteria and fungus from the existing background microorganisms in the environment. A sensitive universal 16S PCR method for bacteria using standard PCR reagents shows a positive result around cycle 28. This represents a detection limit of the system of around 1000 DNA copies, in other words, around 1000 DNA copies are required for reliable detection over background. This amount of DNA is however not found in blood samples of sepsis patients, in particular not in the early stages of disease. A more sensitive detection system is therefore essential, in which the purity and reduction of background microorganism material in the reagents of the reaction is significantly reduced. In an optimal system the sensitivity of such a system would enable detection of one DNA copy. This requires an improvement of a factor of 1000. Significant costs are associated with such technical advances. Due to this reason the diagnostic tests available (for example SepsiFast from Roche) cost approximately 250 EUR per sample, which represents a significant financial hurdle for diagnostic labs and medical systems.

An additional problem during sepsis diagnosis, in particular with PCR based approaches, is the limitation to the detection of particular pathogenic microorganisms. For example the SepsiFast Kit from Roche enables the detection of only a particular subset of potential pathogenic microorganisms. In light of this limitation, a sepsis screening kit that can identify all potential sepsis causes is required.

A third problem with universal pathogen detection is that even when a pathogen is identified, often no information is provided regarding the particular pathogen. In such cases, only a broadband antibiotic therapeutic approach is possible, which may in fact be poorly suited for the particular pathogen in any given case. A sub differentiation of potential pathogens into gram-positive and gram-negative pathogens and subsequently characterization of fungus would provide a vast improvement over the present systems. However, the provision of such a broad universal detection system, in combination with species and bacteria or fungus specific determination represents a significant technological problem. Until the present time no detection system has been disclosed, which enables detection and characterization of gram-positive and gram-negative bacteria in combination with potential detection of fungus.

In comparison to the prior art the present invention provides significant advantages. Current approaches based on PCT determination enable only the detection of bacterial infection. Additionally, the immune response is different from patient to patient and does not always allow a simple diagnosis. Furthermore, no information on the particular type of pathogen is provided, which thereby does not enable provision of an appropriate antibiotic strategy.

The present invention thereby provides a solution to the issues discussed herein with respect to the present disadvantages of methods and other means suitable for detection or identification of the pathogen associated with sepsis, or for diagnosis of sepsis.

One surprising aspect of the invention is that the primers and probes as named herein, for either the 16S or 18S sequence regions, show such a specific binding in such a small sequence region. The primers and/or probes of the invention may in some cases even exhibit overlapping binding regions within the template regions of interest. This represents a surprising aspect of the invention, which is also associated with advantages. In some embodiments, it is advantageous that such a small target/template region is used for the present analysis. This leads to a much larger proportion of all known human pathogens to be detected, as the sequence variation across such a large number of bacterial and fungal species is then minimized through analysis of a smaller target region. The target regions of the present invention (and therefore the oligonucleotides described herein) represent unexpected and advantageous features not previously suggested or thought possible in light of the known art.

A further advantage of the invention is that the present method requires no removal or inactivation of human DNA from the samples before analysis, thereby making the present invention significantly simpler to execute. The known methods in the art are all susceptible to problematic contamination by human DNA, due to poorly designed oligonucleotides, thereby requiring extra removal and/or inactivation steps and a more complicated method. The present method solves this problem by the features disclosed herein.

In one embodiment the invention comprises a method for the diagnosis of sepsis comprising the method for detection and/or identification of a human pathogen and/or genetic material thereof as described herein.

In one embodiment the method for the diagnosis of sepsis of the invention comprises or consists of the following steps:
a) Providing or obtaining a sample, preferably a blood, serum or plasma sample, from a subject exhibiting one or more symptoms of systemic inflammatory response syndrome (SIRS), sepsis, severe sepsis and/or septic shock,
b) Bringing said sample into contact with a mixture of oligonucleotides according to any one of the preceding claims in combination with means for carrying out a nucleic acid amplification reaction, such as amplification reaction reagents,
c) Carrying out a, preferably single, nucleic acid amplification reaction, preferably an RT-PCR, using primers according to SEQ ID NO 1 and 2 and according to SEQ ID NO 9 and 10, or variants thereof as described herein, and using probes according to SEQ ID NO 3, 4, 5, 6, 7, 8, 11, 15 and/or 12, or variants thereof as described herein, and
d) Detecting and evaluating the amplification products generated as a result of said nucleic acid amplification reaction, wherein
e) The detection of the presence of an amplification product and positive signal from a corresponding probe indicates the presence of a human pathogen in the sample analysed and the presence of sepsis.

The present invention also encompasses a corresponding treatment of sepsis as step f) in a subject, preferably human subject, for example after the outcome of the diagnostic method has been obtained from steps a) to d), or after the use of the kit or sets of oligonucleotides described herein, depending on the outcome of the method (diagnosis). The invention also relates to a method for selecting a suitable antibiotic for the treatment of sepsis comprising the methods described herein.

Possible treatments are known to a person skilled in the art. For example, sepsis is treated by prescribing antibiotics. Suitable antibiotics for the various pathogens detected via the present method are known to a skilled person and described herein.

The present invention further relates to a method for the treatment of sepsis in a human subject, comprising:
- having a sample of biological fluid obtained from the subject,
- having an assay conducted on the sample, said assay comprising a multiplex real-time polymerase chain reaction (RT-PCR), wherein said method comprises amplification of PCR products that enable discrimination between bacterial and fungal pathogens, discrimination between gram positive and gram negative bacterial pathogens, and identification of Staphylococcus and/or Candida pathogens if present in said sample, preferably according to one or more of the embodiments described herein,
- Detecting and evaluating the amplification products generated as a result of said nucleic acid amplification reaction, and
- treating the subject for sepsis using one or more antibiotics.

### DETAILED DESCRIPTION OF THE INVENTION

The primers and probes of the present invention, preferably of Table 1, have been especially developed to target a large number of the most important human pathogens associated with bloodstream infection. The present invention thus provides oligonucleotides of from preferably 8 to 50, preferably 12 to 40, preferably 15 to 30 nucleotides long which may be capable of specific binding to a pathogen. These oligonucleotides may be used individually or collectively (in groups or subgroups) in the methods and kits of the present invention.

**Table 1. Description of the sequences of the invention**

| **Description** | **Sequence (5'-3')** | **SEQ ID NO.** |
|---|---|---|
| **Product 144bp; Target 16S** | | |
| Primer Bacteria forward | GCATGGTTGTCGTCAGCTC | 1 |
| Primer Bacteria reverse | GACGTCATCCTCACCTTCCTC | 2 |
| Probe 1 all Bacteria | CCGTAACGAGCGCAACCC | 3 |
| Probe 1 gram-positive | TGCCCAACTGAATGATGGCAAC | 4 |
| Probe 2 gram-positive | AGAGTGCTCAACTGAATGGTAGCAAC | 5 |
| Probe 3 gram-positive | CGCTAGAGTCCTCAACTTAATGGTA | 6 |
| Probe 4 gram-positive | AGTTGACCCCGGCGGTCTCT | 7 |
| Probe 1 Staphylococcus | ACCCTTAAGCTTAGTTGCCTTCA | 8 |

| **Product 139bp; Target 18S** | | |
|---|---|---|
| Primer Fungi forward | GCGCGCAAATTACCCAAT | 9 |
| Primer Fungi reverse | TGTACTCATTCCAATTACGAGACC | 10 |
| Probe 1 all Fungi | CCCTGTATCGTTATTTATTGTCACTACC | 11 |
| Probe 2 all Fungi | CCCTGTATCAGTATTTATTGTCACTACCT | 15 |
| Probe all Candida | TGACATTAAATAACGATACAGGGCC | 12 |

| **Target Sequences** | | |
|---|---|---|
| 16S Target (E. coli Sequence for 16S), ACCESSION AF233451, 1032 to 1172 | | 13 |
| 18S Target (Candida albicans for 18S), ACCESSION AB013586, 409 to 501 | | 14 |

In accordance with the present invention, some of the oligonucleotides of the present invention may be capable of binding (or preferably binding) to a genetic material of one or more pathogen species.

The combinations of primers and/or probes presented herein have not been previously described. In accordance with an embodiment of the invention, detection of the above mentioned bacterial and fungal pathogens may be performed simultaneously. In accordance with a further embodiment of the invention, detection of the above mentioned bacterial and fungal pathogens may be performed in parallel. Of course, if desired, the detection of the above mentioned bacterial and fungal pathogens may be performed separately (i.e., in separate test tubes and/or in separate experiments). A combination of bacterial and fungal amplification systems may also be performed together in a s single tube, for example in a multiplex PCR reaction.

Some aspects of the invention also relate to methods of detection. The methods of detection may be carried out by amplification of the genetic material, by hybridization of the genetic material with oligonucleotides or by a combination of amplification and hybridization. A significant advantage of the present invention is that the amplification step may be performed under similar or uniform amplification conditions for each pathogen species. As such, amplification of each pathogen species may be performed simultaneously. Detection of the genetic material may also advantageously be performed under uniform conditions.

Thus, aspects of the invention relates to methods for detecting and/or identifying a pathogen which may include the steps of contacting a sample comprising or suspected of comprising a pathogen or genetic material originating from the pathogen and adding the oligonucleotide or combination of oligonucleotides under suitable conditions of hybridization, amplification and/or detection.

More specifically, the present invention relates to optimal combinations of amplification primer sequences for efficient multiplex broad-spectrum nucleic acid amplification reaction under uniform conditions of temperature and reagents/buffer solutions for all primer combinations. These combinations may be particularly useful for diagnostic, identification and detection purposes.

Further aspects of the invention relates to combinations of the nucleic acid sequences described herein as well as kits and methods of detection. The present invention aims at developing a nucleic acid-based method, test or kit to detect and/or identify clinically important bacterial and fungal species, preferably those responsible for invasive infections such as sepsis.

The nucleic acid target (genome, gene or gene fragment (e.g., a restriction fragment) of the pathogen) may be in a purified, un-purified form or in an isolated form. The nucleic acid target may be contained within a sample including for example, a biological specimen obtained from a patient, for example a blood, serum or plasma sample, a sample obtained from the environment (soil, objects, etc.), a microbial or tissue culture, a cell line, a preparation of pure or substantially pure pathogens or pathogen mixture etc. In accordance with the present invention, the sample may be obtained from patient having or suspected of having an infection.

### Probes:

Nucleic acid oligonucleotide probes targeting (or binding or hybridizing to) internal regions of the PCR amplicons (products) generated using the amplification primer combinations described herein are encompassed by the present invention. The group of PCR-generated nucleic acid templates is prepared from one or more of the target microbial species mentioned above. These probes can be used for real-time PCR detection (e.g. TaqMan probes, molecular beacons). As used herein, the term "probe" refers to a single-stranded nucleic acid sequence that can be hybridized with a complementary single-stranded target sequence to form a double-stranded molecule (hybrid).

The present invention also features hybridization probes chosen from the regions amplified with the PCR primer pairs described above, i.e., binding within the PCR amplicon amplified by the primers listed herein. These probes can be used for detecting pathogens by either hybridizing to target pathogen nucleic acids amplified with the selected primer pairs or to unamplified target pathogens nucleic acids using signal amplification methods such as ultra-sensitive bio-sensors. When a probe is combined with other probes for simultaneous detection of multiple pathogens, the specificity of the probe should not be substantially affected by the presence of other probes, i.e., it still hybridizes to the target pathogens nucleic acid. Preferably, a probe selected for one pathogen does not hybridize to a nucleic acid from another pathogen. The probes are preferably labelled with different labels that enable simultaneous use and differentiation between each of the labels.

The Real-time PCR (or known as quantitative PCR or qPCR) is a preferred sensitive assay for online monitoring of amplification kinetics. Real-time PCR Probes consist of an oligonucleotide complementary to the amplified probe. The probe is labelled preferably at the 5'-end with the fluorescence donor (for example Fluorescein, HEX or TET) and a few bases downstream or on the 3'-end with a quencher, usually TAMRA. For optimal performance, the quencher's absorbance spectrum should match the fluorophore's emission spectrum as closely as possible. When no complementary sequence is available the fluorescence of the donor is quenched. During PCR amplification the labelled oligonucleotide hybridizes to the target sequence and the 5'-dye is removed by 5'3'-exonuclease activity of Taq. The fluorescence of the donor is no longer quenched and can be measured. The fluorescence intensity is proportional to the amount of PCR-product formed during the early exponential phase of PCR (threshold value).

Modern RT-PCR platforms typically have multiple detection channels enabling flexibility in the choice of probe labels. The fluorescent labels can be selected which are compatible with the detection channels for the qPCR instrument and to ensure the correct filter settings or detection calibration for the instrument.

**Table 2. A number of labels (also known as dyes) and appropriate quenchers are applied:**

| Dye | Max. EX (nm) | Max. EM (nm) | Compatible Quencher |
|---|---|---|---|
| 6-FAMTM | 494 | 515 | BHQ-1, TAMRA |
| JOETM | 520 | 548 | BHQ-1, TAMRA |
| TET | 521 | 536 | BHQ-1, TAMRA |
| Cal Fluor® Gold 540 | 522 | 541 | BHQ-1 |
| HEX | 535 | 555 | BHQ-1, TAMRA |
| Cal Fluor Orange 560 | 540 | 561 | BHQ-1 |
| TAMRATM | 555 | 576 | BHQ-2 |
| Cy3® | 550 | 570 | BHQ-2 |
| Quasar® 570 | 548 | 566 | BHQ-2 |
| Cal Fluor Red 590 | 565 | 588 | BHQ-2 |
| ROXTM | 573 | 6.02 | BHQ-2 |
| Texas Red® | 583 | 603 | BHQ-2 |
| Cy5® | 651 | 674 | BHQ-3 |
| Quasar 670 | 647 | 667 | BHQ-3 |
| Cy5.5® | 675 | 694 | BHQ-3 |

### Primers:

The term "primer" as used herein refers to an oligonucleotide, whether occurring naturally as in a purified restriction digest or produced synthetically, which is capable of acting as a point of initiation of synthesis when placed under conditions in which synthesis of a primer extension product which is complementary to a nucleic acid strand is induced, i.e., in the presence of nucleotides and an inducing agent such as DNA polymerase and at a suitable temperature and pH. The primer is preferably single stranded for maximum efficiency in amplification, but may alternatively be double stranded. If double stranded, the primer is first treated to separate its strands before being used to prepare extension products. Preferably, the primer is an oligodeoxyribonucleotide. The primer must be sufficiently long to prime the synthesis of extension products in the presence of the inducing agent. The exact lengths of the primers will depend on many factors, including temperature, source of primer and use of the method. For example, for diagnostics applications, depending on the complexity of the target sequence, the oligonucleotide primer typically contains 8 to 50, preferably 12-30 or more nucleotides, although it may contain fewer nucleotides.

### Sequence variation:

The oligonucleotide sequence of primers or probes may be derived from either strand of the duplex DNA. The primers or probes may consist of the bases A, G, C, or T or analogs thereof and they may be degenerated at one or more chosen nucleotide position(s) to ensure DNA amplification for potentially all strains of a target bacterial or fungal species. Degenerated primers are primers which have a number of possibilities at mismatch positions in the sequence in order to allow annealing to complementary sequences and amplification of a variety of related sequences. Degeneracies reduce the specificity of the primer(s), meaning mismatch opportunities are greater, and background noise increases; also, increased degeneracy means concentration of the individual primers decreases. Degenerated primers should be carefully designed in order to avoid affecting the sensitivity and/or specificity of the assay. Inosine is a modified base that can bind with any of the regular base (A, T, C or G). Inosine is used in order to minimize the number of degeneracies in an oligonucleotide.

As used herein the term "a 0 to 5 nucleotide addition or deletion at the 5' and/or 3' end of a sequence means that the oligonucleotide or nucleic acid may have a) 0, 1, 2, 3, 4 or 5 additional nucleotides at its 5' end and 0, 1, 2, 3, 4 or 5 nucleotides deleted at its 3' end or b) 0, 1, 2, 3, 4 or 5 additional nucleotide at its 3' end and 0, 1, 2, 3, 4 or 5 nucleotides deleted at its 5' end, c) 0, 1, 2, 3, 4 or 5 additional nucleotide at its 5' end and 0, 1, 2, 3, 4 or 5 additional nucleotides at its 3' end or d) 0, 1, 2, 3, 4 or 5 nucleotide deleted at its 5' end and 0, 1, 2, 3, 4 or 5 nucleotides deleted at its 3' end. Minor changes in the length of the primers and probes described herein may be carried out by a skilled person without undue effort.

In one embodiment the method of the present invention is characterized in that the one or more primer or probe comprises a nucleotide sequence with more than 80% sequence identity, preferably more than 85%, preferably more than 90%, preferably more than 95% sequence identity, to the sequences provided herein, for example, 80, 81, 82, 83, 84, 85, 86, 87, 88, 89, 90, 91, 92, 93, 94, 95, 96, 97, 98, 99% sequence identity. The sequence variant with between 80% and 99% sequence identity is preferably functionally analogous, i. e. sequences that although exhibiting differences in DNA sequence, so that for example the same, fewer or more mismatches may occur between primer/probe and target, exhibit a similar Tm and/or binding specificities for the various pathogens to be detected, so that the function of the primer/probe in the context of the present invention is maintained. Functionally analogous sequences can be tested by one skilled in the art without inventive effort in light of the information provided within the application, for example by testing Tm or hybridization properties in the context of the PCR or other amplification methods described.

### Amplification methods:

One preferred embodiment relates to real time PCR (RT-PCR) or quantitative RT-PCR (qRT-PCR), as it allows the quantification of the amplified target in real-time. The term "real-time PCR" is intended to mean any amplification technique which makes it possible to monitor the progress of an ongoing amplification reaction as it occurs (i.e. in real time). Data is therefore collected during the exponential phase of the PCR reaction, rather than at the end point as in conventional PCR. Measuring the kinetics of the reaction in the early phases of PCR provides distinct advantages over traditional PCR detection. In real-time PCR, reactions are characterized by the point in time during cycling when amplification of a target is first detected rather than the amount of target accumulated after a fixed number of cycles. The higher the starting copy number of the nucleic acid target, the sooner a significant increase in fluorescence is observed. Traditional PCR methods may also be applied, and use separation methods, such as agarose gels, for detection of PCR amplification at the final phase of or end-point of the PCR reaction. For qRT-PCR no post-PCR processing of the unknown DNA sample is necessary as the quantification occurs in real-time during the reaction. Furthermore, an increase in reporter fluorescent signal is directly proportional to the number of amplicons generated.

As the method was designed to use similar experimental conditions, the PCR amplification for each multiplex can be performed using the same thermal cycling profile thereby allowing the amplification of all the nucleic acid targets at the same time in a single apparatus (e.g., ther-mocycler).

Although nucleic acid amplification is often performed by PCR or RT-PCR, other methods exist. Non-limiting examples of such method include quantitative polymerase chain reaction (Q-PCR), ligase chain reaction (LCR), transcription-mediated amplification (TMA), self-sustained sequence replication (3SR), nucleic acid sequence-based amplification (NASBA), strand displacement amplification (SDA), recombinase polymerase amplification (RPA), loop-mediated isothermal amplification (LAMP), helicase-dependent amplification (HDA), helicase-dependent isothermal DNA amplification (tHDA), branched DNA (bDNA), cycling probe technology (CPT), solid phase amplification (SPA), rolling circle amplification technology (RCA), real-time RCA, solid phase RCA, RCA coupled with molecular padlock probe (MPP/RCA), aptamer based RCA (aptamer-RCA), anchored SDA, primer extension preamplification (PEP), degenerate oligonucleotide primed PCR (DOP-PCR), sequence-independent single primer amplification (SISPA), linker-adaptor PCR, nuclease dependent signal amplification (NDSA), ramification amplification (RAM), multiple displacement amplification (MDA), real-time RAM, and whole genome amplification (WGA) (Westin, L. et al., 2000, Nat. Biotechnol. 18:199-204; Notomi, T. et al., 2000, Nucleic Acids Res. 28:e63; Vincent, M. et al., 2004, EMBO reports 5:795-800; Pie-penburg, O. et al., 2006, PLoS Biology 4:E204; Yi, J. et al., 2006, Nucleic Acids Res. 34:e81; Zhang, D. et al., 2006, Clin. Chim. Acta 363:61-70; McCarthy, E. L. et al., 2007, Biosens. Biotechnol. 22:126-1244; Zhou, L. et al., 2007, Anal. Chem. 79:7492-7500; Coskun, S. and Als-madi, O., 2007, Prenat. Diagn. 27:297-302; Biagini, P. et al., 2007, J. Gen. Virol. 88:2629-2701; Gill, P. et al., 2007, Diagn. Microbiol. Infect. Dis. 59:243-249; Lasken, R. S. and Egholm, M., 2003, Trends Biotech. 21:531-535).

### PCR amplification and Multiplex PCR Amplification:

PCR reactions may be performed in mixture containing template genomic DNA preparation obtained for each of the microbial species and diluted at the desired concentration, a buffer suitable for amplification using desired polymerases, primers at a predetermined concentration, dinucleotide triphosphate (dNTPs) mix and DNA polymerase. In order to minimize nucleic acid contamination levels from reagents and solutions, stock solutions may be filtered and solutions may be sterilized and exposed to UV (e.g., using a Spectrolinker™XL-1000 (Spectron-ics Corp.) between 9999 and 40 000 µJ/cm²). UV exposure may be adjusted as described in patent application WO 03087402A1. An internal control designed to monitor amplification efficiency may be added in the multiplex assay(s). Amplification runs may also include no template (negative) control reactions. Amplification may be performed in any thermal cycler. The amplification conditions typically include a step of denaturation of the nucleic acid where suitable denaturation conditions are used, a step of hybridization (annealing) where suitable hybridization conditions are used, a step of extension where suitable extension conditions by the polymerase are used. The amplicons were typically melted between a range of 60° to 95° C. As known by the person skilled in the art, reaction chemistry and cycling conditions may vary and may be optimized for different PCR reagents combinations and thermocycling devices.

### Detection of Amplification Products:

It should also be understood herein that the scope of the invention is not limited to a specific detection technology. Classically, detection of amplified nucleic acids is performed by standard ethidium bromide-stained agarose gel electrophoresis. Briefly, 10 µL of the amplification mixture are resolved by electrophoresis in a 2% agarose gel containing 0.25 µg/mL of ethidium bromide. The amplicons are then visualized under a UV transilluminator. Amplicon size is estimated by comparison with a molecular weight ladder. It is however clear that other method for the detection of specific amplification products, which may be faster and more practical for routine diagnosis, may be used. Such methods may be based on the detection of fluorescence after or during amplification.

One simple method for monitoring amplified DNA is to measure its rate of formation by measuring the increase in fluorescence of intercalating agents such as ethidium bromide or SYBR® Green I (Molecular Probes). If a more specific detection is required, fluorescence-based technologies can monitor the appearance of a specific product during the nucleic acid amplification reaction. The use of dual-labeled fluorogenic probes such as in the TaqMan™ system (Applied Biosystems) which utilizes the 5'-3' exonuclease activity of the Taq polymerase is a good example (Livak K. J. et al., 1995, PCR Methods Appl. 4:357-362). TaqMan™ probes are used during amplification and this "real-time" detection is performed in a closed vessel hence eliminating post-PCR sample handling and consequently preventing the risk of amplicon carryover.

Several other fluorescence-based detection methods can be performed in real-time. Examples of such fluorescence-based methods include the use of adjacent hybridization probes (Wittwer, C. T. et al., 1997, BioTechniques 22:130-138), molecular beacon probes (Tyagi S. and Kramer F. R., 1996, Nat. Biotech. 14:303-308) and scorpion probes (Whitcombe, D. et al., 1999, Nat. Biotechnol. 17:804-807). Adjacent hybridization probes are designed to be internal to the amplification primers. The 3' end of one probe is labelled with a donor fluorophore while the 5' end of an adjacent probe is labelled with an acceptor fluorophore. When the two probes are specifically hybridized in closed proximity (spaced by 1 to 5 nucleotides) the donor fluorophore which has been excited by an external light source emits light that is absorbed by a second acceptor that emit more fluorescence and yields a fluorescence resonance energy transfer (FRET) signal. Molecular beacon probes possess a stem-and-loop structure where the loop is the probe and at the bottom of the stem a fluorescent moiety is at one end while a quenching moiety is at the other end. The molecular beacons undergo a fluorogenic conformational change when they hybridize to their targets hence separating the fluorophore from its quencher. The FRET principle has been used for real-time detection of PCR amplicons in an air thermal cycler equipped with a built-in fluorometer (Wittwer, C. T. et al., 1997, BioTechniques 22:130-138). Apparatus for real-time detection of PCR amplicons are capable of rapid PCR cycling combined with either fluorescent intercalating agents such as SYBR® Green I or FRET detection. Methods based on the detection of fluorescence are particularly promising for utilization in routine diagnosis as they are very simple, rapid and quantitative.

A melting curve analysis may also be used as an alternative to fluorescent techniques. Melting curve analysis is an assessment of the dissociation-characteristics of double-stranded DNA during heating. As the temperature is raised, the double strand begins to dissociate leading to a rise in the absorbance intensity, hyperchromicity. The information gathered can be used to infer the presence and identity of sequence, for example single-nucleotide polymorphisms (SNP). This is due to the fact that G-C base pairing have 3 hydrogen bonds between them while A-T base pairs have only 2. DNA with a higher G-C content, whether because of its source or, as previously mentioned, because of SNPs, will have a higher melting temperature than DNA with a higher A-T content.

Melting curve analysis, for example of PCR products via SYBR Green, other double-strand specific dyes, or probe-based melting curve analysis has become common. The probe-based technique is sensitive enough to detect single-nucleotide polymorphisms (SNP) and can distinguish between homozygous wildtype, heterozygous and homozygous mutant alleles by virtue of the dissociation patterns produced. Without probes, amplicon melting (melting and analysis of the entire PCR product) was not generally successful at finding single base variants through melting profiles. With higher resolution instruments and advanced dyes, amplicon melting analysis of one base variants is now possible with several commercially available instruments. For example: Applied Biosystems 7500 Fast System and the 7900HT Fast Real-Time PCR System, Idaho Technology's LightScanner (the first plate-based high resolution melting device), Qiagen's Rotor-Gene instruments, and Roche's LightCycler 480 instruments.

As used herein, the term "hybridization" refers to the binding of two complementary strands of nucleic acid to form a double-stranded molecule (hybrid). The term binding may be used in place of hybridization in the present invention.

16S ribosomal RNA (or 16S rRNA) is a component of the 30S small subunit of prokaryotic ribosomes. The genes coding for it are referred to as 16S rDNA and may be amplified by the reagents of the present invention. The 16S rRNA gene is used as the standard for classification and identification of microbes, because it is present in most microbes and shows proper changes. Type strains of 16S rRNA gene sequences for most bacteria and archaea are available on public databases such as NCBI. In addition to highly conserved primer binding sites, 16S rRNA gene sequences contain hypervariable regions that can provide species-specific signature sequences useful for bacterial identification. As a result, 16S rRNA gene sequencing has become prevalent in medical microbiology as a rapid and cheap alternative to phenotypic methods of bacterial identification. Although it was originally used to identify bacteria, 16S sequencing was subsequently found to be capable of reclassifying bacteria into completely new species, or even genera. The present invention provides unique combinations of 16S probes, and preferably 16S primer sequences, that enable an enormous number of potentially pathogenic bacteria to be amplified, whilst maintaining a very low background.

18S ribosomal RNA (abbreviated 18S rRNA) is a part of the ribosomal RNA. 18S rRNA is a component of the small eukaryotic ribosomal subunit (40S). 18S rRNA is the structural RNA for the small component of eukaryotic cytoplasmic ribosomes, and thus one of the basic components of all eukaryotic cells. The small subunit (SSU) 18S rRNA gene is one of the most frequently used genes in phylogenetic studies and an important marker for random target polymerase chain reaction (PCR) in environmental biodiversity screening. In general, rRNA gene sequences are easy to access due to highly conserved flanking regions allowing for the use of universal primers. The present invention provides unique combinations of 18S probes, and preferably 18S primer sequences that enable an enormous number of potentially pathogenic fungi to be amplified, whilst maintaining a very low background.

In a preferred embodiment of the invention the method and oligonucleotides provided herein enable discrimination between gram positive and gram negative bacteria. Gram-positive bacteria are those that are stained dark blue or violet by Gram staining. This is in contrast to Gram-negative bacteria, which cannot retain the crystal violet stain, instead taking up the counterstain (safranin or fuchsine) and appearing red or pink. Gram-positive organisms are able to retain the crystal violet stain because of the high amount of peptidoglycan in the cell wall. Gram-positive cell walls typically lack the outer membrane found in Gram-negative bacteria.

In the original bacterial phyla, the Gram-positive organisms made up the phylum Firmicutes, a name now used for the largest group. It includes many well-known genera such as Staphylococcus, Streptococcus, Enterococcus, (which are cocci) and Bacillus, Corynebacterium, Nocardia, Clostridium, Actinobacteria, and Listeria (which are rods and can be remembered by the mnemonic obconical). It has also been expanded to include the Mollicutes, bacteria-like Mycoplasma that lack cell walls and cannot be Gram stained, but are derived from such forms. Actinobacteria are the other major group of Gram-positive bacteria, which typically have a high guanine and cytosine content in their genomes (high G+C group). This contrasts with the Firmicutes, which typically have a low G+C content.

The gram-positive bacteria to be detected may include, but are not limited to, methicillin-susceptible and methicillin-resistant staphylococci (including Staphylococcus aureus, S. epidermidis, S. haemolyticus, S. hominis, S. saprophyticus, and coagulase-negative staphylococci), glycopeptide intermediary- susceptible S. aureus (GISA), vancomycin-resistant Staphylococcus aureus (VRSA), penicillin-susceptible and penicillin-resistant streptococci (including Streptococcus pneumoniae, S. pyogenes, S. agalactiae, S. avium, S. bovis, S. lactis, S. sangius and Streptococci Group C, Streptococci Group G and viridans streptococci), enterococci (including vancomycin-susceptible and vancomycin-resistant strains such asEnterococcus faecalis and E. faecium), Clostridium difficile, C. clostridiiforme, C. innocuum, C. perfringens, C. ramosum, Listeria monocytogenes, Corynebacterium jelkeium, Bifidobacterium spp., Eubacterium aerofaciens, E. lentum, Lactobacillus acidophilus, L. casei, L. plantarum, Lactococcus spp., Leuconostoc spp., Pediococcus, Peptostreptococcus anaeroblus, P. asaccarolyticus, P. magnus, P. micros, P. prevotii, P. productus, Propionibacterium acnes, Actinomyces spp., Moraxella spp. (including M. catarrhalis).

There is a growing need to enhance present diagnostic techniques for infections caused by Gram-negative bacteria, which are difficult to treat because, unlike Gram-positive bacteria, Gram-negative bacteria possess a protective outer membrane consisting of lipopol-ysaacharides. This outer membrane protects the Gram-negative bacteria from antibiotics, dyes, and detergents that would normally damage the inner membrane or cell wall (peptidoglycan). The outer membrane provides these bacteria with resistance to lysozyme and penicillin. While alternative antimicrobial agents such as lysozyme with EDTA and the antibiotics, e.g., ampicillin, chloramphenicol, streptomycin, and nalidixic acid, have been developed to combat the protective outer membrane of some pathogenic Gram-negative microbes, the Gram- negative microbes have been evolving and becoming more immune to existing antimicrobial agents such as antibiotics. As there is a declining pipeline of effective antimicrobial agents, it is imperious to develop more effective detection means to identify Gram-negative microbes, and in the context of the present invention potentially enable discrimination between gram positive and gram negative bacteria.

Antibiotics according to the present invention also encompass potentially the anti-fungal compounds used to treat a diagnosed sepsis of fungi has been detected. The antibiotics commonly applied in the treatment of any given infection, as separated into the classes of pathogen are:
Gram positive coverage: Penicillins, (ampicillin, amoxicillin), penicillinase resistant, (Dicloxacil-lin, Oxacillin), Cephalosporins (1st and 2nd generation), Macrolides (Erythromycin, Clarithro-mycin, Azithromycin), Quinolones (gatifloxacin, moxifloxacin, levofloxacin), Vancomycin, Sulfonamide/trimethoprim, Clindamycin, Tetracyclines, Chloramphenicol, Linezolid, Synercid.
Gram negative coverage: Broad spectrum penicillins (Ticarcillin, clavulanate, piperacillin, tazo-bactam), Cephalosporins (2nd, 3rd, and 4th generation), Aminoglycosides, Macrolides, Azithromycin, Quinolones (Ciprofloxacin), Monobactams (Azetreonam), Sulfonamide/trime-thoprim, Carbapenems (Imipenem), Chloramphenicol.

Pseudomonas coverage: Ciprofloxacin, Aminoglycosides, Some 3rd generation cephalosporins, 4th generation cephalosporins, Broad spectrum penicillins, Carbapenems.

Fungal treatments: Amphotericin B, Fluconazole and other Azoles, itraconazole, voriconazole, posaconazole, ravuconazole, echinocandins, Flucytosine, sordarins, chitin synthetase inhibitors, topoisomerase inhibitors, lipopeptides, pradimycins, Liposomal nystatin, Voriconazole.

Accordingly, the invention comprises the selection of an antibiotic suitable for treatment on the basis of the information obtained by the method described herein.

The invention may encompass the detection of gram-negative microbes. Non-limiting examples of Gram-negative microbes include E. Coli, Salmonella, Shigella, Pseudomonas, Moraxella, Helicobacter, Stenotrophomonas, Bdellovibrio, Legionella, Acinetobacter, spirochaetes, Neisseria gonorrhoeae, Neisseria meningitides, Hemophilus influenzae, Klebsiella pneumoniae, Proteus mirabilis, Enterobacter, Serratia, and any combinations thereof.

A Gram negative bacterium is a bacterium with a cell wall structure that does not retain the methyl violet component of Gram's stain after elution with an organic solvent such as ethyl alcohol. See, e.g., Saltan MJR, Kim KS (1996). Structure, in: Baron's Medical Microbiology (Baron S et al., eds.) (4th ed.). The pink counterstain makes the Gram-negative bacteria appear pink. Gram negative bacteria are characterized by two cellular membranes separated by a periplasmic space. The periplasmic space is external to the inner, cytoplasmic membrane. On the other side of the periplasm is an outer membrane comprising lipopoly saccharide (LPS) and capsular polysaccharide. Porin proteins typically are present on the outer LPS layer. Gramnegative bacteria include, without limitation, Escherichia spp. (e.g., E. Coli); Salmonella spp. (e.g., S. typhimurium); Pseudomonas spp. (e.g., P. aeruginosa); Burkholderia spp.; Neisseria spp. (e.g., N. meningitides, N. gonorrhoeae); Haemophilus spp. (H. influenzae); Shigella spp. Bactericides spp.; Campylobacter spp.; Brucellaspp.; Vibrio spp.; Yersinia spp.; Helicobacter spp.; Calymmatobacterium spp.; Legionella spp.; Leptospira spp.; Borrelia spp., Bordetella spp.; Klebsiella spp. (e.g., K 13655154.2 31 pneumoniae); Treponema spp.; Francisella spp.; Moraxella spp.; Stenotrophomonas spp.; Bdellovibrio spp.; Acinetobacter spp.; Spirochaetes; Proteus spp. (e.g., Proteus microbilis); Enterobacter; Serratia spp. (e.g., S. plymuthica, S. liquefaciens, S. rubidaea, and S. odoriferae); Gardnerella spp., and any combinations thereof. Many of these organisms are known to be pathogenic to animals and/or plants, including mammals such as humans, and can cause diseases and disorders such as enteritis, septicaemia, sepsis, meningitis, enteric fever, pneumonia, epiglottitis, cellulitis, diarrhea and sexually transmitted diseases. For example, Gram- negative cocci include three microorganisms, which cause a sexually transmitted disease (e.g., Neisseria gonorrhoeae), a meningitis (e.g., Neisseria meningitidis), and respiratory symptoms (e.g., Moraxella catarrhalis). Some of Gram-negative bacilli can cause respiratory problems (e.g., Hemophilus influenzae, Klebsiella pneumoniae, Legionella pneumophila, Pseudomonas aeruginosa), urinary problems (e.g., Escherichia coli, Proteus mirabilis, Enterobacter cloacae, Serratia marcescens), and gastrointestinal problems (e.g., Helicobacter pylori, Salmonella enteritidis, Salmonella typhi).Gram-negative bacteria associated with nosocomial infections can also include, but are not limited to, Acinetobacter baumannii, which cause bacteremia, secondary meningitis, sepsis and ventilator-associated pneumonia in intensive-care units of hospital establishments.

In one embodiment the human pathogenic bacteria, either gram positive or gram negative, to be detected or to be tested for may be selected from species of Bordetella, such as Bordetella pertussis, Borrelia, such as Borrelia burgdorferi, Brucella, such as Brucella abortus, Brucella canis, Brucella melitensis or Brucella suis, Campylobacter, such as Campylobacter jejuni, Chlamydia and Chlamydophila, such as Chlamydia pneumonia, Chlamydia trachomatis, Chlamydophila psittaci, Clostridium, such as Clostridium botulinum, Clostridium difficile, Clostridium perfringens, Clostridium tetani, Corynebacterium, such as Corynebacterium diphtheria, Enterococcus, such as Enterococcus faecalis, Enterococcus faecium, Escherichia, such as Escherichia coli, Francisella, such as Francisella tularensis, Haemophilus, such as Haemophilus influenza, Helicobacter, such as Helicobacter pylori, Legionella, such as Legionella pneumophila, Leptospira, such as Leptospira interrogans, Listeria, such as Listeria monocytogenes, Mycobacterium, such as Mycobacterium leprae, Mycobacterium tuberculosis, Mycobacterium ulcerans, Mycoplasma, such as Mycoplasma pneumonia, Neisseria, such as Neisseria gonorrhoeae, Neisseria meningitides, Pseudomonas, such as Pseudomonas aeruginosa, Rickettsia, such as Rickettsia rickettsia, Salmonella, such as Salmonella typhi, Salmonella typhimurium, Shigella, such as Shigella sonnei, Staphylococcus, such as Staphylococcus aureus, Staphylococcus epidermidis, Staphylococcus saprophyticus, Streptococcus, such as Streptococcus agalactiae, Streptococcus pneumonia, Streptococcus pyogenes, Treponema, such as Treponema pallidum, Vibrio, such as Vibrio cholera, Yersinia, such as Yersinia pestis, Yersinia enterocolitica or Yersinia pseudotuberculosis.

Pathogenic fungi are fungi that cause disease in humans or other organisms. Candida species are important human pathogens that are best known for causing opportunist infections in immunocompromised hosts (e.g. transplant patients, AIDS sufferers, cancer patients). Infections are difficult to treat and can be very serious: 30-40% of systemic infections result in death. Aspergillosis is another potential fungal pathogen. Aspergillus can cause disease in three major ways: through the production of mycotoxins; through induction of allergenic responses; and through localized or systemic infections. With the latter two categories, the immune status of the host is pivotal. The most common pathogenic species are Aspergillus fumigatus and Aspergillus flavus. Aspergillus flavus produces aflatoxin which is both a toxin and a carcinogen and which can potentially contaminate foods. Aspergillus fumigatus and Aspergillus clavatus can cause disease. Cryptococcus neoformans can cause disease in humans. Cryptococcus neoformans is the major human and animal pathogen. Cryptococcus laurentii and Cryptococcus albidus have been known to occasionally cause moderate-to-severe disease in human patients with compromised immunity. Cryptococcus gattii is endemic to tropical parts of the continent of Africa and Australia and can cause disease. Histoplasma capsulatum can cause histoplasmosis in humans, dogs and cats. Pneumocystis jirovecii (or Pneumocystis carinii) can cause a form of pneumonia in people with weakened immune systems, such as premature children, the elderly, and AIDS patients. Stachybotrys chartarum or "black mold" can cause respiratory damage and severe headaches.

In one embodiment the human pathogen, either gram positive or gram negative bacteria, or fungus, or other pathogen to be detected or to be tested for may be selected from Acinetobacter baumannii, Klebsiella pneumoniae, Acinetobacter Iwoffii,Listeria monocytogenes, Aeromonas caviae, Morganella morganii,Aeromonas hydrophila, Neisseria gonorrhoeae, Aspergillus flavus,Neisseria meningitidis, Aspergillus nidulans, Pasteurella multocida,Aspergillus niger, Pasteurella pneumotropica, Aspergillus terreus,Propionibacterium acnes, Bacillus anthracis, Proteus mirabillis, Bacillus cereus, Providencia rettgeri, Bacillus subtilis, Pseudomonas aeruginosa,Bacteroides fragilis, Salmonella choleraesuis, Brucella melitensis, Serratia liquefaciens, Burkholderia cepacia, Serratia marcescens, Candida albicans, Staphylococcus aureus, Candida dubliniensis, Staphylococcus epidermidis, Candida glabrata, Staphylococcus haemolyticus, Candida krusei, Staphylococcus hominis, Candida parapsilosis, Staphylococcus saccharolyticus, Candida tropicalis, Staphylococcus warneri,Capnocytophaga canimorsus, Stenotrophomonas maltophilia, Citrobacter braakii, Streptococcus agalactiae, Citrobacter freundii, Streptococcus anginosus, Clostridium perfringens, Streptococcus bovis,Corynebacterium jeikeium, Streptococcus constellatus, Enterobacter aerogenes, Streptococcus dysgalactiae, Enterobacter cloacae,Streptococcus mutans, Enterobacter sakazakii, Streptococcus pneumoniae, Enterococcus faecalis, Streptococcus pyogenes,Enterococcus faecium, Streptococcus salivarius, Escherichia coli,Streptococcus sanguinis, Shigella sp., Streptococcus suis, Gemella haemolysans, Vibrio vulnificus, Gemella morbillorum, Yersinia enterocolitica, Haemophilus influenzae, Yersinia pestis, Kingella kingae,Yersinia pseudotuberculosis and; Klebsiella oxytoca.

### FIGURES

The invention is demonstrated in practical embodiments by the following figures. The embodiments disclosed therein relate to potentially preferred embodiments of the invention and are not intended to limit the scope of the invention.
**Figure 1****:** Amplification curves in the channel 440-488; dye-Atto425 DDQ1-quencher; Detection: Staphylococcus spp. and Candida spp..
**Figure 2****:** Amplification curves in the channel 465-51; dye FAM - quencher IBFQ-ZEN; Detection: Bacteria (sum).
**Figure 3**: Amplification curves in the channel 533-580; dye VIC - quencher IBFQ -ZEN; Detection: Gram-positive bacteria.
**Figure 4****:** Amplification curves in the channel 618-660; dye Cy5 - quencher BHQ2; Detection: Fungi (sum).

### EXAMPLES

The invention is demonstrated in practical embodiments by the following examples. The embodiments disclosed therein relate to potentially preferred embodiments of the invention and are not intended to limit the scope of the invention.

Real time multiplex PCR analysis was carried out using samples comprising bacterial and fungal species, including various human pathogens, as targets. The examples demonstrate that a multiplex approach of the present invention enables the identification and/or detection of a vast number of human pathogenic bacteria, the differentiation between gram-positive and gram-negative bacteria and the detection of staphylococcus as the most common gram-positive pathogen, in addition to the detection of candida fungal species.

### Example 1:

The multiplex approach according to Table 3 was applied to demonstrate the combined sensitivity of the present multiplex reaction, in addition to the stringency with respect to the absence of false-positive results.

A mixture of the five species of pathogens was provided in a test sample at the dilutions provided. Multiplex PCR was carried out using two primer pairs according to SEQ ID No. 1 and 2 (for 16S targets) and SEQ ID No. 9 and 10 (for 18S targets).

The probes according to SEQ ID No. 8 or 12, directed to staphylococcus und candida detection, respectively, tested in combination and separately, were coupled to the 440-488/Atto425 label.

The probe according to SEQ ID No. 3, directed to any given bacterial target, was coupled to the 465-510/FAM label.

The probes according to SEQ ID No. 4, 5, 6 and 7, directed to gram positive bacteria, tested in combination and separately, were coupled to the 533-580/VIC label.

The probes according to SEQ ID No. 11 or 15, directed to fungal targets, tested in combination and separately, were coupled to the 618-660/Cy5 label.

In a preferred embodiment all probes and primers mentioned above were tested in combination in a single multiple PCR reaction.

**Table 3: Experimental approach and results regarding multiplex detection in four channels**

| | | | | ***Staphylococcus spp., Candida spp.*** | **Bacteria Sum** | **Gram + Bacteria** | **Fungus Sum** |
|---|---|---|---|---|---|---|---|
| **Species** | | **MOP** | **Dil.** | **Cp 440-488/Atto425** | **Cp 465-510/FAM** | **Cp 533-580/VIC** | **Cp 618-660/Cy5** |
| NTC | | - | - | - | - | - | - |
| NTC | | - | - | - | - | - | - |
| NTC | | - | - | - | - | - | - |
| NTC | | - | - | - | - | - | - |
| *Escherichia coli* | Bacteria Gram - | 1593 | 1:100 | - | 28,7 | - | - |
| *Staphylococcus aureus* | Bacteria Gram + | 1669 | 1:10 | 22,4 | 24,6 | 21,8 | - |
| *Clostridium difficile* | Bacteria Gram + | 816 | 1:100 | - | 26,4 | 24,8 | - |
| *Aspergillus terreus* | Fungi | 48 | - | - | - | - | 28,2 |
| *Candida glabrata* | Fungi | 2883 | 1:10 | 24,8 | - | - | 21,9 |

The following concentrations and volumes of components were used in the real-time PCR:

| **Component** | **Volume in µl** |
|---|---|
| Water | 8,9 |
| 10 x PCR buffer | 2,2 |
| Deoxyribonucleotide triphosphates mixture, concentration: 2,5mM | 1,8 |
| MgCl₂, concentration: 25mM | 4,4 |
| Primers 0,25 µl, concentration 20pmol/µl | 1,0 |
| Probes 0,16 µl, concentration: 20pmol/µl | 1,6 |
| TAQ-Polymerase, concentration: 10U/µl | 0,1 |
| Total Volume master mix | 20 |
| DNA sample/extract | 5 |
| **Total Volume** | **25** |

The following PCR temperature profile was used:
Initial denaturation: 95°C, 1 min
Cycling: 45 cycles
Denaturation: 95°C, 10 sec
Annealing/extension: 60°C, 15 sec

The PCR reaction was carried out suing a LightCycler 480-II from Roche.

The results can be summarized in table 4.

**Table 4: Schematic summary of test results based on the observations obtained.**

| | **440-488** | **465-510** | **533-580** | **618-660** |
|---|---|---|---|---|
| **Gram negative Bacteria** | - | + | - | - |
| **Gram positive Bacteria (not *Staphylococcus spp.)*** | - | + | + | - |
| ***Staphylococcus* spp.** | + | + | + | - |
| **Fungi (not *Candida spp*.)** | - | - | - | + |
| ***Candida spp.*** | + | - | - | + |
| **invalid** | - | - | - | - |

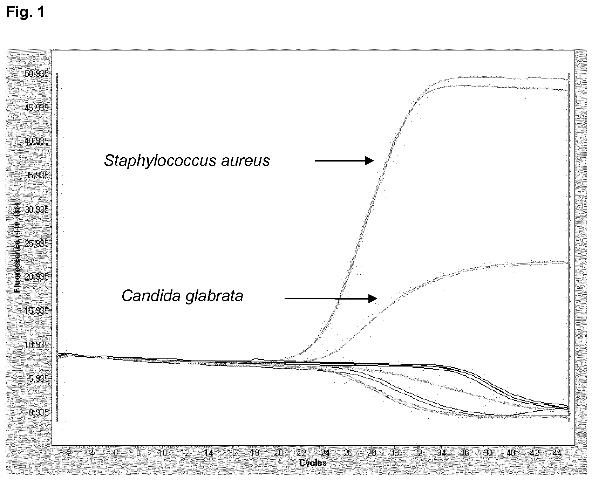

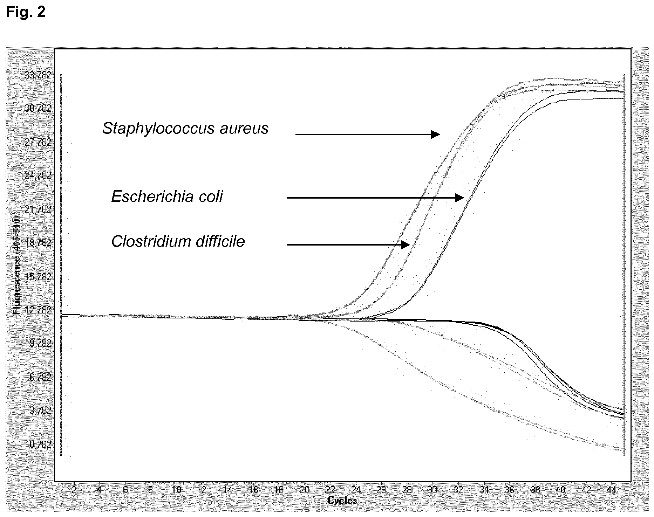

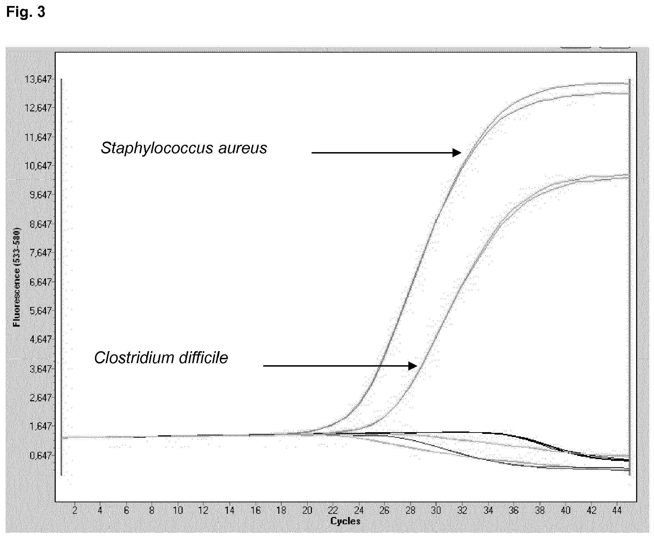

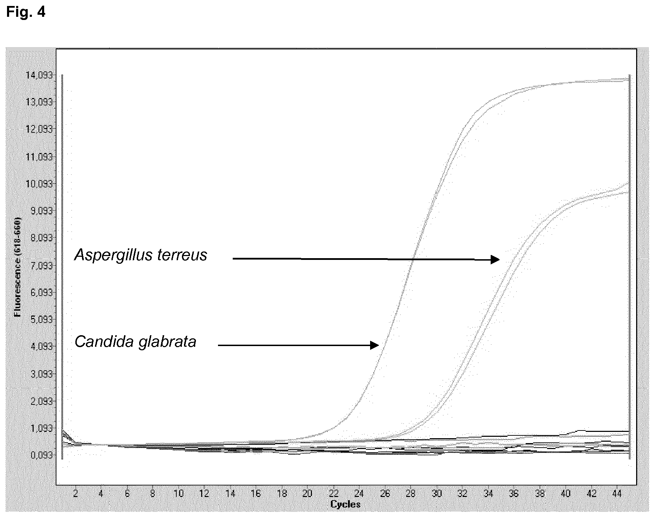

The amplification curves of the four channels analyzed are shown in figures 1 to 4.

### Example 2:

This approach described in example 1 was carried out for a number of pathogens in order to demonstrate the broad spectrum of microbes capable of detection by the present method.

**Table 5: List of microbes analyzed and results with respect to amplification.**

| **Species** | **440488/ Atto425** | **465510/ FAM** | **533580/ VIC** | **618660/ Cy5** | **Results** |
|---|---|---|---|---|---|
| *Acinetobacter baumannii* | - | **+** | - | - | Gram - Bacteria |
| *Arcobacter butzleri* | - | **+** | - | - | Gram - Bacteria |
| *Aeromonas caviae* | - | **+** | - | - | Gram - Bacteria |
| *Aeromonas hydrophila* | - | **+** | - | - | Gram - Bacteria |
| *Areomonas sobria* | - | **+** | - | - | Gram - Bacteria |
| *Aggregatibacter actino-mycetemcommitans* | - | **+** | - | - | Gram - Bacteria |
| *Aggregatibacter aphro-philus* | - | **+** | - | - | Gram - Bacteria |
| *Alcaligenes faecalis* | - | **+** | - | - | Gram - Bacteria |
| *Bordetella bronchiseptica* | - | **+** | - | - | Gram - Bacteria |
| *Bordetella parapertussis* | - | **+** | - | - | Gram - Bacteria |
| *Bordetella pertussis* | - | **+** | - | - | Gram - Bacteria |
| *Burkholderia cepacia* | - | **+** | - | - | Gram - Bacteria |
| *Campylobacter jejuni* | - | **+** | - | - | Gram - Bacteria |
| *Campylobacter coli* | - | **+** | - | - | Gram - Bacteria |
| *Campylobacter fetus ssp. Fetus* | - | **+** | - | - | Gram - Bacteria |
| *Campylobacter rectus* | - | **+** | - | - | Gram - Bacteria |
| *Campylobacter upsali-ensis* | - | **+** | - | - | Gram - Bacteria |
| *Cedecca neteri* | - | **+** | - | - | Gram - Bacteria |
| *Citrobacter freundii* | - | **+** | - | - | Gram - Bacteria |
| *Citrobacter koseri* | - | **+** | - | - | Gram - Bacteria |
| *Cronobacter dublinensis* | - | **+** | - | - | Gram - Bacteria |
| *Cronobacter muytjensii* | - | **+** | - | - | Gram - Bacteria |
| *Cronobacter sakazakii* | - | **+** | - | - | Gram - Bacteria |
| *Cronobacter turicensis* | - | **+** | - | - | Gram - Bacteria |
| *Edwardsiella tarda* | - | **+** | - | - | Gram - Bacteria |
| *Enterobacter aerogenes* | - | **+** | - | - | Gram - Bacteria |
| *Enterobacter amnige-nus* | - | **+** | - | - | Gram - Bacteria |
| *Enterobacter cloacae* | - | **+** | - | - | Gram - Bacteria |
| *Escherichia coli* | - | **+** | - | - | Gram - Bacteria |
| *Eubacterium rectale* | - | **+** | - | - | Gram - Bacteria |
| *Fluoribacter dumoffii* | - | **+** | - | - | Gram - Bacteria |
| *Haemophilus influenzae* | - | **+** | - | - | Gram - Bacteria |
| *Hafnia alvei* | - | **+** | - | - | Gram - Bacteria |
| *Klebsiella oxytoca* | - | **+** | - | - | Gram - Bacteria |
| *Klebsiella pneumoniae* | - | **+** | - | - | Gram - Bacteria |
| *Legionella anisa* | - | **+** | - | - | Gram - Bacteria |
| *Legionella bozemanae* | - | **+** | - | - | Gram - Bacteria |
| *Legionella gormanii* | - | **+** | - | - | Gram - Bacteria |
| *Legigionella long-beachae* | - | **+** | - | - | Gram - Bacteria |
| *Legionella maceachernii* | - | **+** | - | - | Gram - Bacteria |
| *Legionella micdadei* | - | **+** | - | - | Gram - Bacteria |
| *Legionella pneumophila* | - | **+** | - | - | Gram - Bacteria |
| *Moraxella (Branha-mella) catarrhalis* | - | **+** | - | - | Gram - Bacteria |
| *Plesiomonas* | - | **+** | - | - | Gram - Bacteria |
| *Plesimonas shigelloides* | - | **+** | - | - | Gram - Bacteria |
| *Proteus mirabilis* | - | **+** | - | - | Gram - Bacteria |
| *Proteus vulgaris* | - | **+** | - | - | Gram - Bacteria |
| *Providencia alcalifa-ciens* | - | **+** | - | - | Gram - Bacteria |
| *Pseudomonas aeruginosa* | - | **+** | - | - | Gram - Bacteria |
| *Pseudomonas flu-orescens* | - | **+** | - | - | Gram - Bacteria |
| *Pseudomonas putida* | - | **+** | - | - | Gram - Bacteria |
| *Pseudomonas stutzeri* | - | **+** | - | - | Gram - Bacteria |
| *Salmonella anatum* | - | **+** | - | - | Gram - Bacteria |
| *Salmonella bonpori* | - | **+** | - | - | Gram - Bacteria |
| *Salmonella bovismorbif-icans* | - | **+** | - | - | Gram - Bacteria |
| *Salmonella branden-burg* | - | **+** | - | - | Gram - Bacteria |
| *Salmonella enterica* | - | **+** | - | - | Gram - Bacteria |
| *Salmonella enteritidis* | - | **+** | - | - | Gram - Bacteria |
| *Salmonella infantis* | - | **+** | - | - | Gram - Bacteria |
| *Salmonella kentucky* | - | **+** | - | - | Gram - Bacteria |
| *Salmonella typhimurium* | - | **+** | - | - | Gram - Bacteria |
| *Serratia liqufaciens* | - | **+** | - | - | Gram - Bacteria |
| *Serratia marcescens* | - | **+** | - | - | Gram - Bacteria |
| *Shigella boydii* | - | **+** | - | - | Gram - Bacteria |
| *Shigella sonnei* | - | **+** | - | - | Gram - Bacteria |
| *Stenotrophomonas maltophilia* | - | **+** | - | - | Gram - Bacteria |
| *Vibrio alginolyticus* | - | **+** | - | - | Gram - Bacteria |
| *Vibrio campbelli* | - | **+** | - | - | Gram - Bacteria |
| *Vibrio carchariae* | - | **+** | - | - | Gram - Bacteria |
| *Vibrio fluvialis* | - | **+** | - | - | Gram - Bacteria |
| *Vibrio furnissii* | - | **+** | - | - | Gram - Bacteria |
| *Vibrio hollisae* | - | **+** | - | - | Gram - Bacteria |
| *Vibrio cincinnatiensis* | - | **+** | - | - | Gram - Bacteria |
| *Vibrio metschrikovii* | - | **+** | - | - | Gram - Bacteria |
| *Vibrio natriegens* | - | **+** | - | - | Gram - Bacteria |
| *Vibrio parahaemolyticus* | - | **+** | - | - | Gram - Bacteria |
| *Vibrio proteolyticus* | - | **+** | - | - | Gram - Bacteria |
| *Yersinia enterocolitica* | - | **+** | - | - | Gram - Bacteria |
| *Acholeplasma laidlawii* | - | **+** | **+** | - | Gram + Bacteria |
| *Bacillus cereus* | - | **+** | **+** | - | Gram + Bacteria |
| *Bacillus mycoides* | - | **+** | **+** | - | Gram + Bacteria |
| *Bacillus cytotoxis* | - | **+** | **+** | - | Gram + Bacteria |
| *Bacillus pseudomycoides* | - | **+** | **+** | - | Gram + Bacteria |
| *Bacillus subtilis* | - | **+** | **+** | - | Gram + Bacteria |
| *Bacillus thuringiensis* | - | **+** | **+** | - | Gram + Bacteria |
| *Bacillus weihenstaphanensis* | - | **+** | **+** | - | Gram + Bacteria |
| *Clostridium botulinum* | - | **+** | **+** | - | Gram + Bacteria |
| *Clostridium botulinum A* | - | **+** | **+** | - | Gram + Bacteria |
| *Clostridium botulinum F* | - | **+** | **+** | - | Gram + Bacteria |
| *Clostridium botulinum E* | - | **+** | **+** | - | Gram + Bacteria |
| *Clostridium botulinum B* | - | **+** | **+** | - | Gram + Bacteria |
| *Clostridium bowmanii* | - | **+** | **+** | - | Gram + Bacteria |
| *Clostridium difficile Toxin A*/*B* | - | **+** | **+** | - | Gram + Bacteria |
| *Clostridium estertheti-cum* | - | **+** | **+** | - | Gram + Bacteria |
| *Clostridium friporis* | - | **+** | **+** | - | Gram + Bacteria |
| *Clostridium hystolyticum* | - | **+** | **+** | - | Gram + Bacteria |
| *Clostridium lacusfryxel-lense* | - | **+** | **+** | - | Gram + Bacteria |
| *Clostridium perfringens* | - | **+** | **+** | - | Gram + Bacteria |
| *Clostridium septicum* | - | **+** | **+** | - | Gram + Bacteria |
| *Clostridium sordelli* | - | **+** | **+** | - | Gram + Bacteria |
| *Clostridium sporopenes* | - | **+** | **+** | - | Gram + Bacteria |
| *Clostridium tyrobutyricum* | - | **+** | **+** | - | Gram + Bacteria |
| *Enterococcus casseluflavus* | - | **+** | **+** | - | Gram + Bacteria |
| *Enterococcus durans* | - | **+** | **+** | - | Gram + Bacteria |
| *Enterococcus fecalis* | - | **+** | **+** | - | Gram + Bacteria |
| *Enterococcus faecium* | - | **+** | **+** | - | Gram + Bacteria |
| *Lactobacillus casei* | - | **+** | **+** | - | Gram + Bacteria |
| *Lactobacillus plantarum* | - | **+** | **+** | - | Gram + Bacteria |
| *Lactobacillus johnsonii* | - | **+** | **+** | - | Gram + Bacteria |
| *Lactobacillus rhamnosus* | - | **+** | **+** | - | Gram + Bacteria |
| *Lactobacillus sakei subsp. Sakei* | - | **+** | **+** | - | Gram + Bacteria |
| *Lactococcus lactis* | - | **+** | **+** | - | Gram + Bacteria |
| *Listeria innova* | - | **+** | **+** | - | Gram + Bacteria |
| *Listeria innocua* | - | **+** | **+** | - | Gram + Bacteria |
| *Listeria ivanovii* | - | **+** | **+** | - | Gram + Bacteria |
| *Listeria grayi* | - | **+** | **+** | - | Gram + Bacteria |
| *Listeria monocytogenes* | - | **+** | **+** | - | Gram + Bacteria |
| *Listeria seeliperi* | - | **+** | **+** | - | Gram + Bacteria |
| *Listeria welshimeri* | - | **+** | **+** | - | Gram + Bacteria |
| *Mycoplasma gallisepticum* | - | **+** | - | - | Gram - Bacteria |
| *Mycoplasma genitalium* | - | **+** | - | - | Gram - Bacteria |
| *Mycoplasma orale* | - | **+** | - | - | Gram - Bacteria |
| *Mycoplasma pneumoniae* | - | **+** | - | - | Gram - Bacteria |
| *Mycoplasma synoviae* | - | **+** | - | - | Gram - Bacteria |
| *Pediococcus acidilactici* | - | **+** | **+** | - | Gram + Bacteria |
| *Staphylococcus aureus* | **+** | **+** | **+** | - | Gram +/ *Staphylococcus spp.* |
| *Staphylococcus capitis* | **+** | **+** | **+** | - | Gram +/ *Staphylococcus spp.* |
| *Staphylococcus cohnii* | **+** | **+** | **+** | - | Gram +/ *Staphylococcus spp.* |
| *Staphylococcus epidermidis* | **+** | **+** | **+** | - | Gram +/ *Staphylococcus spp.* |
| *Staphylococcus haemolyticus* | **+** | **+** | **+** | - | Gram +/ *Staphylococcus spp.* |
| *Staphylococcus hominis* | **+** | **+** | **+** | - | Gram +/ *Staphylococcus spp.* |
| *Stapylococcus saprophyticus* | **+** | **+** | **+** | - | Gram +/ *Staphylococcus spp.* |
| *Staphylococcus warneri* | **+** | **+** | **+** | - | Gram +/ *Staphylococcus spp.* |
| *Stapylococcus xylosus* | **+** | **+** | **+** | - | Gram +/ *Staphylococcus spp.* |
| *Streptococcus agalactiae* | - | **+** | **+** | - | Gram + Bacteria |
| *Streptococcus anginosus* | - | **+** | **+** | - | Gram + Bacteria |
| *Streptococcus criceti* | - | **+** | **+** | - | Gram + Bacteria |
| *Streptococcus equisimilis* | - | **+** | **+** | - | Gram + Bacteria |
| *Streptococcus gallolyticus* | - | **+** | **+** | - | Gram + Bacteria |
| *Streptococcus mutans* | - | **+** | **+** | - | Gram + Bacteria |
| *Streptococcus oralis* | - | **+** | **+** | - | Gram + Bacteria |
| *Streptococcus parasanguinis* | - | **+** | **+** | - | Gram + Bacteria |
| *Streptococcus pneumoniae* | - | **+** | **+** | - | Gram + Bacteria |
| *Streptococcus pyogenes* | - | **+** | **+** | - | Gram + Bacteria |
| *Streptococcus sanguinis* | - | **+** | **+** | - | Gram + Bacteria |
| *Streptococcus salivarius* | - | **+** | **+** | - | Gram + Bacteria |
| *Streptococcus thermophilus* | - | **+** | **+** | - | Gram + Bacteria |
| *Streptococcus uberis* | - | **+** | **+** | - | Gram + Bacteria |
| *Bifidobacterium animalis subsp. Lactis* | - | **+** | **+** | - | Gram + Bacteria |
| *Corynebacterium diphtheriae* | - | **+** | **+** | - | Gram + Bacteria |
| *Corynebacterium xerosis* | - | **+** | **+** | - | Gram + Bacteria |
| *Gardnerella vaginalis* | - | **+** | **+** | - | Gram + Bacteria |
| *Micrococcus luteus* | - | **+** | **+** | - | Gram + Bacteria |
| *Mycobacterium avium subsp paratuberculosis* | - | **+** | **+** | - | Gram + Bacteria |
| *Mycobacterium bovis* | - | **+** | **+** | - | Gram + Bacteria |
| *Nocardia brasiliensis* | - | **+** | **+** | - | Gram + Bacteria |
| *Propionibacterium acnes* | - | **+** | **+** | - | Gram + Bacteria |
| *Streptomyces griseus* | - | **+** | **+** | - | Gram + Bacteria |
| *Streptomyces mobaraensis* | - | **+** | **+** | - | Gram + Bacteria |
| *Borrelia afzellii* | - | **+** | - | - | Gram - Bacteria |
| *Borrelia burgdorferi* | - | **+** | - | - | Gram - Bacteria |
| *Helicobacter pylori* | - | **+** | - | - | Gram - Bacteria |
| *Treponema denticola* | - | **+** | - | - | Gram - Bacteria |
| *Chlamydia trachomatis* | - | **+** | - | - | Gram - Bacteria |
| *Chlamydophila pneumoniae* | - | **+** | - | - | Gram - Bacteria |
| *Chlamydophila psittaci* | - | **+** | - | - | Gram - Bacteria |
| *Fusobacterium nucleatum subsp. Nucleatum* | - | **+** | - | - | Gram - Bacteria |
| *Fusobacterium necrophorum* | - | **+** | - | - | Gram - Bacteria |
| *Bacteroides fragilis* | - | **+** | - | - | Gram - Bacteria |
| *Bacteroides ovatus* | - | **+** | - | - | Gram - Bacteria |
| *Bacteroides thetaiotaomicron* | - | **+** | - | - | Gram - Bacteria |
| *Bacteroides vulpatus* | - | **+** | - | - | Gram - Bacteria |
| *Ornithobacterium* | - | **+** | - | - | Gram - Bacteria |
| *Porphyromonas gingivalis* | - | **+** | - | - | Gram - Bacteria |
| *Prevotella intermedia* | - | **+** | - | - | Gram - Bacteria |
| *Prevotella melaninogenica* | - | **+** | - | - | Gram - Bacteria |
| *Tanerella forsythia* | - | **+** | - | - | Gram - Bacteria |
| *Arthroderma benhamiae* | - | - | - | **+** | Fungi |
| *Aspergillus fumigatus* | - | - | - | **+** | Fungi |
| *Aspergillus niger* | - | - | - | **+** | Fungi |
| *Aspergillus terrus* | - | - | - | **+** | Fungi |
| *Aspergillus versicolor* | - | - | - | **+** | Fungi |
| *Candida albicans* | **+** | - | - | **+** | Fungi/ *Candida spp.* |
| *Candida glabrata* | **+** | - | - | **+** | Fungi/ *Candida spp.* |
| *Candida krusei* | **+** | - | - | **+** | Fungi/ *Candida spp.* |
| *Candida parapsilosis* | **+** | - | - | **+** | Fungi/ *Candida spp.* |
| *Candida tropicalis* | **+** | - | - | **+** | Fungi/ *Candida spp.* |
| *Cunninghamella spec.* | - | - | - | **+** | Fungi |
| *Crytococcus neoformans* | - | - | - | **+** | Fungi |
| *Fusarium culmorum* | - | - | - | **+** | Fungi |
| *Microsporum canis* | - | - | - | **+** | Fungi |
| *Microsporum gypseum* | - | - | - | **+** | Fungi |
| *Mucor spec.* | - | - | - | **+** | Fungi |
| *Scopulariopsis brevicaulis* | - | - | - | **+** | Fungi |
| *Trichophyton interdigitale* | - | - | - | **+** | Fungi |
| *Trichphyton rubrum* | - | - | - | **+** | Fungi |

## Claims

1. An in vitro method for the detection and/or identification of a human pathogen and/or genetic material thereof comprising subjecting a sample comprising or suspected of comprising a human pathogen and/or genetic material thereof to a multiplex real-time polymerase chain reaction (RT-PCR), wherein said method comprises amplification of PCR products that enable:
a) discrimination between bacterial and fungal pathogens,
b) discrimination between gram positive and gram negative bacterial pathogens, and
c) identification of Staphylococcus and/or Candida pathogens if present in said sample,
wherein the method is carried out in a single PCR reaction using oligonucleotide primers and probes that hybridize with a 16S rRNA sequence of a bacterial pathogen and a 18S rRNA sequence of a fungal pathogen, wherein said probes exhibit distinct fluorescent labels that may be identified and differentiated from one another on the basis of emitting light at different wavelengths, and
wherein the oligonucleotide primers that hybridize with a 16S rRNA sequence hybridize with and prime the amplification of a sequence that corresponds to SEQ ID No. 13 with reference to the Escherichia coli 16S sequence, and the oligonucleotide primers that hybridize with an 18S rRNA sequence hybridize with and prime the amplification a sequence that corresponds to SEQ ID No. 14 with reference to the Candida albicans 18S sequence.

2. Method according to the preceding claim, wherein one set of primers comprising one forward primer and one reverse primer is employed for amplification of a 16S rRNA sequence of a bacterial pathogen and one set of primers comprising one forward primer and one reverse primer is employed for amplification of an 18S rRNA sequence of a fungal pathogen.

3. Method according to any one of the preceding claims, wherein the oligonucleotides that hybridize with a 16S rRNA sequence of a bacterial pathogen comprise the oligonucleotides of a) and b), or c), wherein:
a) Oligonucleotide probes, each of whose sequence consists of a sequence according to SEQ ID NO 3, 4, 5, 6, 7 or 8, respectively, or oligonucleotides of a complementary sequence, wherein said probes are labelled such to be distinguished from each other,
b) Oligonucleotide primers, each of whose sequence consists of a sequence according to SEQ ID NO 1 or 2, respectively, or oligonucleotides of a complementary sequence,
c) Oligonucleotides of a) and b) that comprise a 0 to 5 nucleotide addition or deletion at their 5' and/or 3' end, or oligonucleotides of a complementary sequence.

4. Method according to any one of the preceding claims, wherein the oligonucleotides that hybridize with an 18S rRNA sequence of a fungal pathogen comprise the oligonucleotides of a) and b), or c), wherein:
a) Oligonucleotide probes, each of whose sequence consists of a sequence according to SEQ ID NO 11, 15 or 12, respectively, or oligonucleotides of a complementary sequence, wherein said probes are labelled such to be distinguished from each other,
b) Oligonucleotide primers, each of whose sequence consists of a sequence according to SEQ ID NO 9 or 10, respectively,
c) Oligonucleotides of a) and b) that comprise a 0 to 5 nucleotide addition or deletion at their 5' and/or 3' end, or oligonucleotides of a complementary sequence.

5. Method according to any one of the preceding claims, wherein amplification products are generated as a result of said nucleic acid amplification reaction and the detecting and/or evaluating of said products comprises:
a) detecting an amplification product, preferably of 144bp, produced using primers according to SEQ ID NO 1 and 2,
b) detecting an amplification product, preferably of 92bp, produced using primers according to SEQ ID NO 9 and 10, and
c) detecting one or more labels associated with one or more probes according to SEQ ID NO 3, 4, 5, 6, 7, 8, 11, 15 and/or 12, wherein
i. detection of a label associated with SEQ ID NO 3 indicates the presence of bacteria,
ii. detection of a label associated with SEQ ID NO 4 indicates the presence of gram-positive bacteria,
iii. detection of a label associated with SEQ ID NO 5 indicates the presence of gram-positive bacteria,
iv. detection of a label associated with SEQ ID NO 6 indicates the presence of gram-positive bacteria,
v. detection of a label associated with SEQ ID NO 7 indicates the presence of gram-positive bacteria,
vi. detection of a label associated with SEQ ID NO 8 indicates the presence of a staphylococcus species,
vii. detection of a label associated with SEQ ID NO 11 indicates the presence of fungi,
viii. detection of a label associated with SEQ ID NO 15 indicates the presence of fungi, and/or
ix. detection of a label associated with SEQ ID NO 12 indicates the presence of a Candida species.

6. Method according to any one of the preceding claims for the detection and/or identification of a pathogen in a sample obtained from a subject exhibiting one or more symptoms of systemic inflammatory response syndrome (SIRS), sepsis, severe sepsis and/or septic shock.

7. Method according to any one of the preceding claims, comprising treatment of the RT-PCR reaction reagents to remove or neutralise genetic material capable of producing background (unwanted) nucleic acid amplification.

8. Method for the diagnosis of sepsis comprising carrying out the method according to any one of the preceding claims.

9. Method for the selection of an antibiotic for the treatment of a subject diagnosed with sepsis, comprising carrying out the method according to any one of the preceding claims, and selection of an antibiotic based on the human pathogen identified by said method.

10. An oligonucleotide set, comprising the oligonucleotides of a) or b), and c) or d), wherein:
a) Oligonucleotides, each of whose sequence consists of a sequence according to SEQ ID NO 1, 2, 3, 4, 5, 6, 7 or 8, respectively, or oligonucleotides of a complementary sequence,
b) Oligonucleotides of a) that comprise a 0 to 5 nucleotide deletion at their 5' and/or 3' end, or oligonucleotides of a complementary sequence,
c) Oligonucleotides, each of whose sequence consists of a sequence according to SEQ ID NO 9, 10, 11, 15 or 12, respectively, or oligonucleotides of a complementary sequence,
d) Oligonucleotides of c) that comprise a 0 to 5 nucleotide deletion at their 5' and/or 3' end, or oligonucleotides of a complementary sequence.

11. Kit suitable for carrying out the method of any one of claims 1 to 9, comprising
a) the oligonucleotide set according to claim 10, and
b) amplification reagents, such as one or more fluorescent labels, dNTPs, buffers and/or enzymes.

## Patentansprüche

1. In-vitro-Verfahren zum Nachweis und/oder zur Identifizierung eines Humanpathogens und/oder genetischen Materials davon, umfassend das Einbringen einer Probe, die ein Humanpathogen und/oder genetisches Material davon umfasst oder bei der ein Verdacht darauf besteht, dass sie dies umfasst, in eine Multiplex-Echtzeit-Polymerase-Kettenreaktion (RT-PCR), wobei das Verfahren die Amplifikation von PCR-Produkten umfasst, die Folgendes ermöglichen:
a) Unterscheidung zwischen bakteriellen und Pilzpathogenen,
b) Unterscheidung zwischen grampositiven und gramnegativen bakteriellen Pathogenen, und
c) Identifizierung von Staphylococcus- und/oder Candida-Pathogenen, falls in der Probe vorliegend,
wobei das Verfahren in einer einzigen PCR-Reaktion durchgeführt wird, die Oligonukleotid-Primer und Sonden verwendet, die mit einer 16S-rRNA-Sequenz eines bakteriellen Pathogens und einer 18S-rRNA-Sequenz eines Pilzpathogens hybridisieren, wobei die Sonden unterschiedliche Fluoreszenzmarkierungen zeigen, die basierend darauf, dass sie Licht mit verschiedenen Wellenlängen aussenden, identifiziert und voneinander unterschieden werden können, und
wobei die Oligonukleotid-Primer, die mit einer 16S-rRNA-Sequenz hybridisieren, mit einer Sequenz hybridisieren und deren Amplifikation primen, die SEQ ID No. 13 unter Bezugnahme auf die Escherichia-coli-16S-Sequenz entspricht, und die Oligonukleotid-Primer, die mit einer 18S-rRNA-Sequenz hybridisieren, mit einer Sequenz hybridisieren und deren Amplifikation primen, die SEQ ID No. 14 unter Bezugnahme auf die Candida-albicans-18S-Sequenz entspricht.

2. Verfahren nach dem vorstehenden Anspruch, wobei ein Satz Primer, der einen Vorwärts-Primer und einen Rückwärts-Primer umfasst, für die Amplifikation einer 16S-rRNA-Sequenz eines bakteriellen Pathogens eingesetzt wird und ein Satz Primer, der einen Vorwärts-Primer und einen Rückwärts-Primer umfasst, für die Amplifikation einer 18S-rRNA-Sequenz eines Pilzpathogens eingesetzt wird.

3. Verfahren nach einem der vorstehenden Ansprüche, wobei die Oligonukleotide, die mit einer 16S-rRNA-Sequenz eines bakteriellen Pathogens hybridisieren, die Oligonukleotide aus a) und b), oder c) umfassen, wobei:
a) Oligonukleotid-Sonden, deren Sequenz jeweils aus einer Sequenz gemäß SEQ ID NO 3, 4, 5, 6, 7 beziehungsweise 8 besteht, oder Oligonukleotide einer komplementären Sequenz, wobei die Sonden so markiert sind, dass sie voneinander unterschieden werden können,
b) Oligonukleotid-Primer, deren Sequenz jeweils aus einer Sequenz gemäß SEQ ID NO 1 beziehungsweise 2 besteht, oder Oligonukleotide einer komplementären Sequenz,
c) Oligonukleotide aus a) und b), die eine Addition oder Deletion von 0 bis 5 Nukleotiden an ihrem 5'- und/oder 3'-Ende umfassen, oder Oligonukleotide einer komplementären Sequenz.

4. Verfahren nach einem der vorstehenden Ansprüche, wobei die Oligonukleotide, die mit einer 18S-rRNA-Sequenz eines Pilzpathogens hybridisieren, die Oligonukleotide aus a) und b), oder c) umfassen, wobei:
a) Oligonukleotid-Sonden, deren Sequenz jeweils aus einer Sequenz gemäß SEQ ID NO 11, 15 beziehungsweise 12 besteht, oder Oligonukleotide einer komplementären Sequenz, wobei die Sonden so markiert sind, dass sie voneinander unterschieden werden können,
b) Oligonukleotid-Primer, deren Sequenz jeweils aus einer Sequenz gemäß SEQ ID NO 9 beziehungsweise 10 besteht,
c) Oligonukleotide aus a) und b), die eine Addition oder Deletion von 0 bis 5 Nukleotiden an ihrem 5'- und/oder 3'-Ende umfassen, oder Oligonukleotide einer komplementären Sequenz.

5. Verfahren nach einem der vorstehenden Ansprüche, wobei Amplifikationsprodukte als Ergebnis der Nukleinsäureamplifikationsreaktion erzeugt werden und das Nachweisen und/oder Auswerten dieser Produkte Folgendes umfasst:
a) Nachweisen eines Amplifikationsproduktes, vorzugsweise von 144bp, das unter Verwendung von Primern gemäß SEQ ID NO 1 und 2 erzeugt wurde,
b) Nachweisen eines Amplifikationsproduktes, vorzugsweise von 92bp, das unter Verwendung von Primern gemäß SEQ ID NO 9 und 10 erzeugt wurde, und
c) Nachweisen einer oder mehrerer Markierungen in Verbindung mit einer oder mehreren Sonden gemäß SEQ ID NO 3, 4, 5, 6, 7, 8, 11, 15 und/oder 12, wobei
i. der Nachweis einer Markierung in Verbindung mit SEQ ID NO 3 auf das Vorliegen von Bakterien hinweist,
ii. der Nachweis einer Markierung in Verbindung mit SEQ ID NO 4 auf das Vorliegen von grampositiven Bakterien hinweist,
iii. der Nachweis einer Markierung in Verbindung mit SEQ ID NO 5 auf das Vorliegen von grampositiven Bakterien hinweist,
iv. der Nachweis einer Markierung in Verbindung mit SEQ ID NO 6 auf das Vorliegen von grampositiven Bakterien hinweist,
v. der Nachweis einer Markierung in Verbindung mit SEQ ID NO 7 auf das Vorliegen von grampositiven Bakterien hinweist,
vi. der Nachweis einer Markierung in Verbindung mit SEQ ID NO 8 auf das Vorliegen einer Staphylococcus-Spezies hinweist,
vii. der Nachweis einer Markierung in Verbindung mit SEQ ID NO 11 auf das Vorliegen von Pilzen hinweist,
viii. der Nachweis einer Markierung in Verbindung mit SEQ ID NO 15 auf das Vorliegen von Pilzen hinweist, und/oder
ix. der Nachweis einer Markierung in Verbindung mit SEQ ID NO 12 auf das Vorliegen einer Candida-Spezies hinweist.

6. Verfahren nach einem der vorstehenden Ansprüche zum Nachweis und/oder zur Identifizierung eines Pathogens in einer Probe, die von einem Individuum gewonnen wurde, das ein oder mehrere Symptome eines systemischen inflammatorischen Response-Syndroms (SIRS), von Sepsis, schwerer Sepsis und/oder eines septischen Schocks zeigt.

7. Verfahren nach einem der vorstehenden Ansprüche, umfassend die Behandlung der Reaktionsreagenzien der RT-PCR, um genetisches Material, das in der Lage ist, eine (unerwünschte) Hintergrund-Nukleinsäureamplifikation zu erzeugen, zu entfernen oder zu neutralisieren.

8. Verfahren zur Diagnose von Sepsis, umfassend das Durchführen des Verfahrens nach einem der vorstehenden Ansprüche.

9. Verfahren zur Auswahl eines Antibiotikums zur Behandlung eines Individuums, bei dem Sepsis diagnostiziert wurde, umfassend das Durchführen des Verfahrens nach einem der vorstehenden Ansprüche und Auswahl eines Antibiotikums basierend auf dem Humanpathogen, das durch das Verfahren identifiziert wurde.

10. Ein Satz Oligonukleotide, umfassend die Oligonukleotide aus a) oder b), und c) oder d), wobei:
a) Oligonukleotide, deren Sequenz jeweils aus einer Sequenz gemäß SEQ ID NO 1, 2, 3, 4, 5, 6, 7 beziehungsweise 8 besteht, oder Oligonukleotide einer komplementären Sequenz,
b) Oligonukleotide aus a), die eine Deletion von 0 bis 5 Nukleotiden an ihrem 5'- und/oder 3'-Ende umfassen, oder Oligonukleotide einer komplementären Sequenz,
c) Oligonukleotide, deren Sequenz jeweils aus einer Sequenz gemäß SEQ ID NO 9, 10, 11, 15 beziehungsweise 12 besteht, oder Oligonukleotide einer komplementären Sequenz,
d) Oligonukleotide aus c), die eine Deletion von 0 bis 5 Nukleotiden an ihrem 5'- und/oder 3'-Ende umfassen, oder Oligonukleotide einer komplementären Sequenz.

11. Ein Kit, geeignet zum Durchführen des Verfahrens nach einem der Ansprüche 1 bis 9, umfassend
a) den Satz Oligonukleotide nach Anspruch 10 und
b) Amplifikationsreagenzien, wie etwa eine oder mehrere Fluoreszenzmarkierungen, dNTPs, Puffer und/oder Enzyme.

## Revendications

1. Procédé in vitro pour la détection et/ou l'identification d'un pathogène humain et/ou d'un matériel génétique de celui-ci comprenant le soumission d'un échantillon comprenant ou suspecté de comprendre un pathogène humain et/ou un matériel génétique de celui-ci à une amplification en chaîne par polymérase en temps réel (RT-PCR) multiplex, dans lequel ledit procédé comprend l'amplification de produits PCR qui permettent :
a) une distinction entre des pathogènes bactériens et fongiques,
b) une distinction entre des pathogènes bactériens à Gram-positif et à Gram-négatif, et
c) l'identification de pathogènes Staphylococcus et/ou Candida s'ils sont présents dans ledit échantillon,
dans lequel le procédé est effectué dans une unique réaction PCR en utilisant des amorces et des sondes oligonucléotidiques qui s'hybrident à une séquence d'ARNr 16S d'un pathogène bactérien et à une séquence d'ARNr 18S d'un pathogène fongique, dans lequel lesdites sondes présentent des marqueurs fluorescents distincts qui peuvent être identifiés et différenciés les uns des autres sur la base d'une émission de lumière à différentes longueurs d'onde, et
dans lequel les amorces oligonucléotidiques qui s'hybrident à une séquence d'ARNr 16S s'hybrident à et amorcent l'amplification d'une séquence qui correspond à SEQ ID No. 13 en se référant à la séquence 16S d'Escherichia coli, et les amorces oligonucléotidiques qui s'hybrident à une séquence d'ARNr 18S s'hybrident à et amorcent l'amplification d'une séquence qui correspond à SEQ ID No. 14 en se référant à la séquence 18S de Candida albicans.

2. Procédé selon la revendication précédente, dans lequel un ensemble d'amorces comprenant une amorce sens et une amorce antisens est utilisé pour l'amplification d'une séquence d'ARNr 16S d'un pathogène bactérien et un ensemble d'amorces comprenant une amorce sens et une amorce antisens est utilisé pour l'amplification d'une séquence d'ARNr 18S d'un pathogène fongique.

3. Procédé selon l'une quelconque des revendications précédentes, dans lequel les oligonucléotides qui s'hybrident à une séquence d'ARNr 16S d'un pathogène bactérien comprennent les oligonucléotides de a) et b), ou c), dans lequel :
a) des sondes oligonucléotidiques, dont chaque séquence consiste en une séquence selon SEQ ID NO 3, 4, 5, 6, 7 ou 8, respectivement, ou des oligonucléotides d'une séquence complémentaire, dans lequel lesdites sondes sont marquées de manière à être distinguées les unes des autres,
b) des amorces oligonucléotidiques, dont chaque séquence consiste en une séquence selon SEQ ID NO 1 ou 2, respectivement, ou des oligonucléotides d'une séquence complémentaire,
c) des oligonucléotides de a) et b) qui comprennent une addition ou délétion de 0 à 5 nucléotides à leurs extrémités 5' et/ou 3', ou des oligonucléotides d'une séquence complémentaire.

4. Procédé selon l'une quelconque des revendications précédentes, dans lequel les oligonucléotides qui s'hybrident à une séquence d'ARNr 18S d'un pathogène fongique comprennent les oligonucléotides de a) et b), ou c), dans lequel :
a) des sondes oligonucléotidiques, dont chaque séquence consiste en une séquence selon SEQ ID NO 11, 15 ou 12, respectivement, ou des oligonucléotides d'une séquence complémentaire, dans lequel lesdites sondes sont marquées de manière à être distinguées les unes des autres,
b) des amorces oligonucléotidiques, dont chaque séquence consiste en une séquence selon SEQ ID NO 9 ou 10, respectivement,
c) des oligonucléotides de a) et b) qui comprennent une addition ou délétion de 0 à 5 nucléotides à leurs extrémités 5' et/ou 3', ou des oligonucléotides d'une séquence complémentaire.

5. Procédé selon l'une quelconque des revendications précédentes, dans lequel des produits d'amplification sont générés à la suite de ladite réaction d'amplification d'acide nucléique et la détection et/ou l'évaluation desdits produits comprennent :
a) la détection d'un produit d'amplification, de préférence de 144 pb, produit en utilisant les amorces selon SEQ ID NO 1 et 2,
b) la détection d'un produit d'amplification, de préférence de 92 pb, produit en utilisant les amorces selon SEQ ID NO 9 et 10, et
c) la détection d'un ou de plusieurs marqueurs associés à une ou plusieurs sondes selon SEQ ID NO 3, 4, 5, 6, 7, 8, 11, 15 et/ou 12, dans lequel
i. la détection d'un marqueur associé à SEQ ID NO 3 indique la présence de bactéries,
ii. la détection d'un marqueur associé à SEQ ID NO 4 indique la présence de bactéries à Gram-positif,
iii. la détection d'un marqueur associé à SEQ ID NO 5 indique la présence de bactéries à Gram-positif,
iv. la détection d'un marqueur associé à la SEQ ID NO 6 indique la présence de bactéries à Gram-positif,
v. la détection d'un marqueur associé à SEQ ID NO 7 indique la présence de bactéries à Gram-positif,
vi. la détection d'un marqueur associé à SEQ ID NO 8 indique la présence d'une espèce de staphylocoque,
vii. la détection d'un marqueur associé à SEQ ID NO 11 indique la présence de champignons,
viii. la détection d'un marqueur associé à SEQ ID NO 15 indique la présence de champignons, et/ou
ix. la détection d'un marqueur associé à SEQ ID NO 12 indique la présence d'une espèce de Candida.

6. Procédé selon l'une quelconque des revendications précédentes pour la détection et/ou l'identification d'un pathogène dans un échantillon provenant d'un sujet présentant un ou plusieurs symptômes du syndrome de réponse inflammatoire systémique (SIRS), d'un sepsis, d'un sepsis sévère et/ou d'un choc septique.

7. Procédé selon l'une quelconque des revendications précédentes, comprenant le traitement des réactifs de la réaction RT-PCR pour éliminer ou neutraliser un matériel génétique capable de produire une amplification d'acide nucléique d'arrière-plan (non souhaitée).

8. Procédé de diagnostic d'un sepsis comprenant la mise en œuvre du procédé selon l'une quelconque des revendications précédentes.

9. Procédé de sélection d'un antibiotique pour le traitement d'un sujet diagnostiqué avec un sepsis, comprenant la mise en œuvre du procédé selon l'une quelconque des revendications précédentes, et la sélection d'un antibiotique sur la base du pathogène humain identifié par ledit procédé.

10. Ensemble d'oligonucléotides, comprenant les oligonucléotides de a) ou b), et c) ou d), dans lequel :
a) des oligonucléotides, dont chaque séquence consiste en une séquence selon SEQ ID NO 1, 2, 3, 4, 5, 6, 7 ou 8, respectivement, ou des oligonucléotides d'une séquence complémentaire,
b) des oligonucléotides de a) qui comprennent une délétion de 0 à 5 nucléotides à leurs extrémités 5' et/ou 3', ou des oligonucléotides d'une séquence complémentaire,
c) des oligonucléotides, dont chaque séquence consiste en une séquence selon SEQ ID NO 9, 10, 11, 15 ou 12, respectivement, ou des oligonucléotides d'une séquence complémentaire,
d) des oligonucléotides de c) qui comprennent une délétion de 0 à 5 nucléotides à leurs extrémités 5' et/ou 3', ou des oligonucléotides d'une séquence complémentaire.

11. Kit approprié pour la mise en œuvre du procédé selon l'une quelconque des revendications 1 à 9, comprenant
a) l'ensemble d'oligonucléotides selon la revendication 10, et
b) des réactifs d'amplification, tels qu'un ou plusieurs marqueurs fluorescents, dNTP, tampons et/ou enzymes.


### SEQUENCE LISTING

<110> Mergemeier, Steffen
<120> METHOD FOR THE DETECTION OF SEPSIS
<130> XII 2004/14WO
<150> EP 14192644.4
   <151> 2014-11-11
<150> EP 14178228.4
   <151> 2014-07-23
<160> 15
<170> PatentIn version 3.5
<210> 1
   <211> 19
   <212> DNA
   <213> Artificial Sequence
<220>
   <223> primer
<400> 1
   gcatggttgt cgtcagctc 19
<210> 2
   <211> 21
   <212> DNA
   <213> Artificial Sequence
<220>
   <223> primer
<400> 2
   gacgtcatcc tcaccttcct c 21
<210> 3
   <211> 18
   <212> DNA
   <213> Artificial Sequence
<220>
   <223> probe
<400> 3
   ccgtaacgag cgcaaccc 18
<210> 4
   <211> 22
   <212> DNA
   <213> Artificial Sequence
<220>
   <223> probe
<400> 4
   tgcccaactg aatgatggca ac 22
<210> 5
   <211> 26
   <212> DNA
   <213> Artificial Sequence
<220>
   <223> probe
<400> 5
   agagtgctca actgaatggt agcaac 26
<210> 6
   <211> 25
   <212> DNA
   <213> Artificial Sequence
<220>
   <223> probe
<400> 6
   cgctagagtc ctcaacttaa tggta 25
<210> 7
   <211> 20
   <212> DNA
   <213> Artificial Sequence
<220>
   <223> probe
<400> 7
   agttgacccc ggcggtctct 20
<210> 8
   <211> 23
   <212> DNA
   <213> Artificial Sequence
<220>
   <223> probe
<400> 8
   acccttaagc ttagttgcct tca 23
<210> 9
   <211> 18
   <212> DNA
   <213> Artificial Sequence
<220>
   <223> primer
<400> 9
   gcgcgcaaat tacccaat 18
<210> 10
   <211> 24
   <212> DNA
   <213> Artificial Sequence
<220>
   <223> primer
<400> 10
   tgtactcatt ccaattacga gacc 24
<210> 11
   <211> 28
   <212> DNA
   <213> Artificial Sequence
<220>
   <223> probe
<400> 11
   ccctgtatcg ttatttattg tcactacc 28
<210> 12
   <211> 25
   <212> DNA
   <213> Artificial Sequence
<220>
   <223> probe
<400> 12
   tgacattaaa taacgataca gggcc 25
<210> 13
   <211> 140
   <212> DNA
   <213> Escherichia coli
<400> 13
   gcatggctgt cgtcagctcg tgttgtgaaa tgttgggtta agtcccgcaa cgagcgcaac 60
   ccttatcctt tgttgccagc ggtccggccg ggaactcaaa ggagactgcc agtgataaac 120
   tggaggaagg tggggatgac 140
<210> 14
   <211> 92
   <212> DNA
   <213> Candida albicans
<400> 14
   gcgcgcaaat tacccaatcc cgattcaggg gaggtagtga caataaataa cgatacaggg 60
   cccttttggg tcttgtaatt ggaatgagta ca 92
<210> 15
   <211> 29
   <212> DNA
   <213> Artificial Sequence
<220>
   <223> probe
<400> 15
   ccctgtatca gtatttattg tcactacct 29